# EUROPÄISCHE PATENTSCHRIFT

(11) **EP 0 772 603 B1**
(45) Veröffentlichungstag und Bekanntmachung des Hinweises auf die Patenterteilung: **12.06.2002**
(21) Anmeldenummer: 95926898.8
(22) Anmeldetag: 14.07.1995
(51) Int. Cl.: C07D 239/56, C07D 401/12, C07D 239/38, C07D 239/46, C07D 239/60, A61K 31/505

(54) **SUBSTITUIERTE PYRIMIDINVERBINDUNGEN UND DEREN VERWENDUNG**
SUBSTITUTED PYRIMIDINE COMPOUNDS AND THEIR USE
COMPOSES SUBSTITUES DE PYRIMIDINE ET LEUR UTILISATION

(30) Priorität: 15.07.1994 DE 4425143
(43) Veröffentlichungstag der Anmeldung: 14.05.1997
(73) Patentinhaber: BASF AKTIENGESELLSCHAFT, 67056 Ludwigshafen (DE)
(72) Erfinder: HELLENDAHL, Beate, D-67105 Schifferstadt (DE); LANSKY, Annegret, D-64297 Darmstadt (DE); MUNSCHAUER, Rainer, Shrewsbury, MA 01545 (US); BIALOJAN, Siegfried, D-68723 Oftersheim (DE); UNGER, Liliane, D-67065 Ludwigshafen (DE); TESCHENDORF, Hans-Jürgen, D-67373 Dudenhofen (DE); WICKE, Karsten, D-67122 Altrip (DE); DRESCHER, Karla, D-69221 Dossenheim (DE)
(74) Vertreter: Kinzebach, Werner, Dr.
(86) Internationale Anmeldenummer: EP9502784
(87) Internationale Veröffentlichungsnummer: WO9602519

(56) Entgegenhaltungen:
- DE-A- 1 942 405
- DE-A- 1 946 172
- DE-A- 2 139 082
- DE-A- 2 258 561

## Beschreibung

Die Erfindung betrifft substituierte Pyrimidinverbindungen und die Verwendung derartiger Verbindungen. Die erwähnten Verbindungen besitzen wertvolle therapeutische Eigenschaften und sind insbesondere zur Behandlung von Erkrankungen brauchbar, die auf Dopamin-D₃-Liganden ansprechen.

Verbindungen der hier in Rede stehenden Art mit physiologischer Aktivität sind bereits teilweise bekannt. So beschreiben DE 21 39 082 und DE 22 58 561 basisch substituierte Pyrimidinderivate bzw. Pyrimidonderivate als Arzneimittel zur Senkung des Blutdrucks. Diese Pyrimidin- bzw. Pyrimidonderivate besitzen die Formeln: 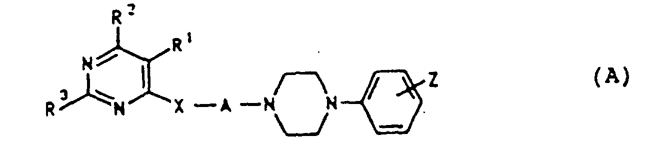 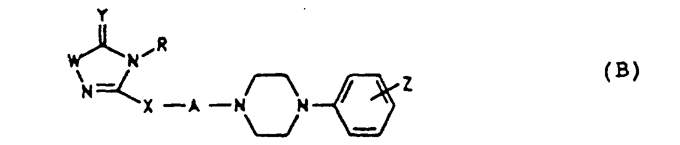 worin in (A) X unter anderem ein Schwefelatom bedeutet, A eine C₁-C₆-Alkylengruppe bedeutet und R¹, R², R³ und Z für verschiedene Substituenten stehen. In (B) stehen X und Y für ein Sauerstoffoder Schwefelatom, A für eine C₂-C₆-Alkylengruppe und R und Z für verschiedene Substituenten.

Die DE-A-19 46 172 beschreibt Verbindungen der Formel 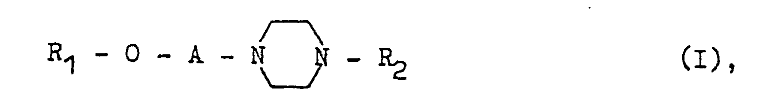 worin
- R1: unter anderem für 4-Propyl-6-methylpyrimidinyl-(2)- steht,
- A: für C₂-C₆-Alkylen steht und
- R²: für Phenyl oder Pyridyl steht, das durch Niedrigalkyl, Niedrigalkoxy, Cl oder Br substituiert ist. Diese Verbindungen besitzen α-sympatikolytische Wirkung und zeigen einen sedativen, Blutdruck senkenden und Gefäß erweiternden Effekt.

Die US-A-5,075,308 offenbart Verbindungen der Formel 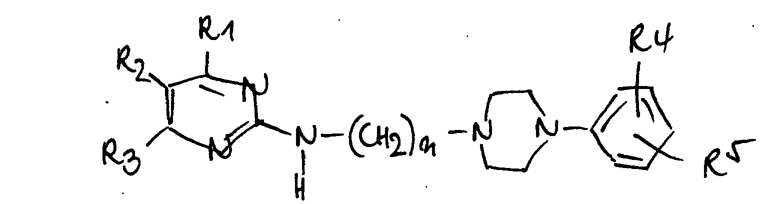 Worin R₁ bis R₅ und n bestimmte Bedentungen haben. Die Verbindungen werden zur Behandlung von Harnwegsverstopfungen eingesetzt.

Neuronen erhalten ihre Informationen unter anderem über G-Protein-gekoppelte Rezeptoren. Es gibt zahlreiche Substanzen, welche ihre Wirkung über diese Rezeptoren ausüben. Eine davon ist Dopamin.

Es liegen gesicherte Erkenntnisse über die Anwesenheit von Dopamin und dessen physiologische Funktion als Neurotransmitter vor. Auf Dopamin ansprechende Zellen stehen im Zusammenhang mit der

Etiologie von Schizophrenie und der Parkinson'schen Krankheit. Die Behandlung dieser und anderer Erkrankungen erfolgt mit Arzneimitteln, die mit den Dopaminrezeptoren in Wechselwirkung treten.

Bis 1990 waren zwei Subtypen von Dopaminrezeptoren pharmakologisch klar definiert, nämlich die D₁ und D₂-Rezeptoren.

Sokoloff et al., Nature 1990, 347 : 146 - 151, hat einen dritten Subtyp gefunden, nämlich die D₃-Rezeptoren. Sie werden hauptsächlich im limbischen System exprimiert. Strukturell unterscheiden sich die D₃-Rezeptoren von den D₁- und D₂-Rezeptoren in etwa der Hälfte der Aminosäurereste.

Die Wirkung von Neuroleptika wurde im allgemeinen ihrer Affinität zu den D₂-Rezeptoren zugeschrieben. Neuere Rezeptorbindungsstudien haben dies bestätigt. Danach besitzen die meisten Dopaminantagonisten, wie Neuroleptika, hohe Affinität zu den D₂-Rezeptoren, aber nur geringe Affinität zu den D₃-Rezeptoren.

Überraschenderweise wurde nun gefunden, daß bestimmte Pyrimidinverbindungen eine hohe Affinität zum Dopamin-D₃-Rezeptor und eine geringe Affinität zum D₂-Rezeptor aufweisen. Es handelt sich somit um selektive D₃-Liganden.

Gegenstand der vorliegenden Erfindung ist daher die Verwendung der Pyrimidinverbindungen der allgemeinen Formel I: 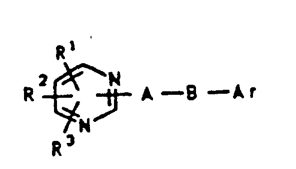 worin
- A: für eine C₁-C₁₈-Alkylengruppe steht, die gegebenenfalls mindestens eine Gruppe umfaßt, die ausgewählt ist unter O, S, NR⁴, CONR⁴, NR⁴CO, COO, OCO und einer Doppel- oder einer Dreifachbindung,
- B: für 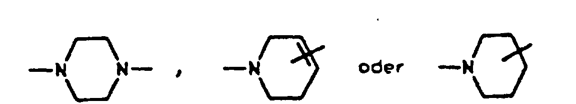 steht,
- R¹, R², R³: unabhängig voneinander ausgewählt sind unter H, Halogen, OR⁴, NR⁴R⁵, SR⁴, CF₃, CN, CO₂R⁴ und C₁-C₈-Alkyl, das gegebenenfalls durch OH, OC₁-C₈-Alkyl oder Halogen substituiert ist,
- R4: für H, C₁-C₈-Alkyl, das gegebenenfalls durch OH, OC₁-C₈-Alkyl oder Halogen substituiert ist, steht,
- R⁵: die für R⁴ angegebenen Bedeutungen besitzt oder für COR⁴ oder CO₂R⁴ steht;
- Ar: für Phenyl, Pyridyl, Pyrimidyl oder Triazinyl steht,
wobei Ar gegebenenfalls ein bis vier Substituenten aufweisen kann, die unabhängig voneinander ausgewählt sind unter OR⁵, C₁-C₈-Alkyl, C₂-C₆-Alkenyl, C₂-C₆-Alkinyl, Halogen, CN, CO₂R⁴, NO₂, SO₂R⁴, SO₃R⁴, NR⁴R⁵, SO₂NR⁴R⁵, SR⁴, CF₃, CHF₂, einem 5- oder 6-gliedrigen carbocyclischen, aromatischen oder nicht-aromatischen Ring und einem 5- oder 6-gliedrigen heterocyclischen aromatischen oder nicht-aromatischen Ring mit 1 bis 3 Heteroatomen, die ausgewählt sind unter O, S und N,
wobei der Ring gegebenenfalls substituiert sein kann durch C₁-C₈-Alkyl, Hal, OC₁-C₈-Alkyl, OH, NO₂, CF₃ und
wobei
Ar gegebenenfalls auch mit einem carbocyclischen oder heterocyclischen Ring der oben definierten Art kondensiert sein kann,
und der Salze davon mit physiologisch verträglichen Säuren zur Herstellung eines pharmazeutischen Mittels zur Behandlung von Erkrankungen, die auf Dopamin-D₃-Rezeptorantagonisten bzw. -agonisten ansprechen.

Die Erfindung betrifft auch die Pyrimidinverbindungen der Formel IA, IB, IC bzw. ID 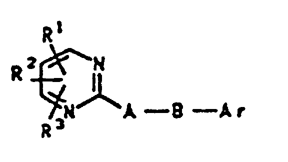 worin
A, B, Ar, R¹, R² und R³ die in den Ansprüchen 1 bis 15 angegebenen Bedeutungen besitzen, und die Salze davon mit physiologisch verträglichen Säuren,
ausgenommen die Verbindungen der Formel 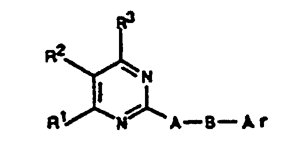 worin R¹ für OH oder SH steht, R² und R³ unabhängig voneinander für H, C₁-C₆-Alkyl, OC₁-C₆-Alkyl, SC₁-C₆-Alkyl, CO₂H, OH, SH, NR⁴R⁵ oder Halogen stehen, wobei R⁴ und R⁵ für H oder C₁-C₆-Alkyl stehen, A für SC₁-C₆-Alkylen, B für 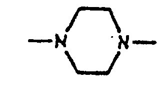 steht und Ar für Phenyl steht, das gegebenenfalls einen oder mehrere Substituenten aufweist, die ausgewählt sind unter C₁-C₄-Alkyl, OC₁-C₄-Alkyl, SC₁-C₄-Alkyl, NO₂, CF₃, F, Cl oder Br.

Bei den erfindungsgemäß zur Anwendung kommenden Verbindungen handelt es sich um selektive Dopamin-D₃-Rezeptor-Liganden, die regioselektiv im limbischen System angreifen und auf Grund ihrer geringen Affinität zum D₂-Rezeptor nebenwirkungsärmer als die klassischen Neuroleptika sind, bei denen es sich um D₂-Rezeptorantagonisten handelt. Die Verbindungen sind daher zur Behandlung von Erkrankungen brauchbar, die auf Dopamin-D₃-Rezeptorantagonisten bzw. -agonisten ansprechen, z.B. zur Behandlung von Erkrankungen des zentralen Nervensystems insbesondere Schizophrenie, Depressionen, Neurosen und Psychosen. Außerdem sind sie zur Behandlung von Schlafstörungen, Übelkeit und als Antihistaminika brauchbar.

Im Rahmen der vorliegenden Erfindung besitzen die nachfolgenden Ausdrücke die anschließend angegebenen Bedeutungen:

Alkyl (auch in Resten wie Alkoxy, Alkylamino etc) bedeutet eine geradkettige oder verzweigte Alkylgruppe mit 1 bis 8 Kohlenstoffatomen, vorzugsweise 1 bis 6 Kohlenstoffatomen und insbesondere 1 bis 4 Kohlenstoffatomen. Die Alkylgruppe kann einen oder mehrere Substituenten aufweisen, die unabhängig voneinander ausgewählt sind unter OH und OC₁-C₈-Alkyl.

Beispiele für eine Alkylgruppe sind Methyl, Ethyl, n-Propyl, i-Propyl, n-Butyl, iso-Butyl, t-Butyl, etc.

Alkylen steht für geradkettige oder verzweigte Reste mit vorzugsweise 2 bis 15 Kohlenstoffatomen, besonders bevorzugt 3 bis 10 Kohlenstoffatomen.

Die Alkylengruppen können gegebenenfalls wenigstens eine der oben angegebenen Gruppen umfassen. Diese kann - ebenso wie die erwähnte Doppel- oder Dreifachbindung - in der Alkylenkette an beliebiger Stelle oder am Ende der Kette angeordnet sein, so, daß sie die Kette mit dem Pyrimidinrest verbindet. Letzteres ist bevorzugt. Wenn die Alkylengruppe eine Doppel- oder Dreifachbindung umfaßt, besitzt sie mindestens drei Kohlenstoffatome in der Kette.

Halogen bedeutet, F, Cl, Br, I und insbesondere Cl, Br, I.

Vorzugsweise stehen R¹, R² und R³ unabhängig voneinander für H, C₁-C₈-Alkyl, NR⁴R⁵, SR⁴ oder OR⁴, wobei R⁴ und R⁵ unabhängig voneinander für H oder C₁-C₈-Alkyl stehen.

Vorzugsweise weist der Rest Ar einen oder zwei Substituenten auf, die unabhängig voneinander ausgewählt sind unter OR⁵, C₁-C₈-Alkyl, Hal, CN, CO₂R⁴, NO₂, SO₂R⁴, SO₃R⁴, NR⁴R⁵, SO₂NR⁴R⁵, SR⁴, CF₃, CHF₂, einem 5- oder 6-gliedrigen carbocyclischen, aromatischen oder nichtaromatischen Ring und einem 5- bis 6-gliedrigen heterocyclischen, aromatischen oder nichtaromatischen Ring mit 1 bis 3 Heteroatomen, die ausgewählt sind unter 0, S und N, wobei der Ring gegebenenfalls substituiert sein kann durch C₁-C₈-Alkyl, Hal, OC₁-C₉-Alkyl, OH, NO₂, CF₃ und wobei Ar gegebenenfalls auch mit einem carbocyclischen oder heterocyclischen Ring der oben definierten Art kondensiert sein kann.

Wenn der Rest Ar einen oder zwei Substituenten aufweist, befinden diese sich vorzugsweise in m-Stellung. Vorzugsweise sind sie unabhängig ausgewählt unter Halogen, CF₃, CHF₂, CN, NO₂, OR⁴, NR⁴R⁵, C₁-C₈-Alkyl, OC₁-C₈-Alkyl, Phenyl und SR⁴, wobei R⁴ und R⁵ für H oder C₁ - C₈-Alkyl stehen. Wenn einer der Substituenten für C₁-C₈-Alkyl steht, ist eine verzweigte Gruppe und insbesondere Isopropyl oder t-Butyl bevorzugt.

Ar weist vorzugsweise wenigstens einen Substituenten auf und steht insbesondere für 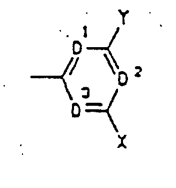 worin D¹, D² und D³ unabhängig voneinander für CR oder N stehen und R, X und Y für H oder die oben bzw. nachfolgend angegebenen Bedeutungen stehen.

Vorzugsweise steht Ar für gegebenenfalls substituiertes Phenyl, 2-, 3- oder 4-Pyridinyl oder 2-, 4(6)- oder 5- Pyrimidinyl.

Wenn einer der Substituenten des Restes Ar für einen 5- oder 6-gliedrigen heterocyclischen Ring steht, so handelt es sich beispielsweise um einen Pyrrolidin-, Piperidin-, Horpholin-, Piperazin-, Pyridin-, 1,4-Dihydropyridin-, Pyrimidin-, Triazin-, Pyrrol-, Thiophen-, Thiazol-, Imidazol-, Oxazol-, Isoxazol-, Pyrazol-, oder Thiadiazolrest.

Wenn einer der Substituenten des Restes Ar für einen carbocyclischen Rest steht, handelt es sich insbesondere um einen Phenyl-, Cyclopentyl- oder Cyclohexylrest.

Wenn Ar mit einen carbocyclischen oder heterocyclischen Rest kondensiert ist, handelt es sich insbesondere um einen Naphthalin-, Di- oder Tetrahydronaphthalin-, Chinolin-, Di- oder Tetrahydrochinolin, Indol-, Dihydroindol-, Benzimidazol-, Benzothiazol-, Benzothiadiazol-, Benzopyrrol- oder Benzotriazolrest.

Eine bevorzugte Ausführung ist die Verwendung von Verbindungen der Formel I, worin A für C₁-C₁₀-Alkylen steht, das gegebenenfalls wenigstens eine Gruppe umfasst, die ausgewählt ist unter O, S, NR⁴, Cyclohexylen und einer Doppel- oder Dreifachbindung.

Besonders bevorzugt sind Verbindungen der Formel IA, IB oder IC, worin A für C₃-C₁₀-Alkylen steht, das gegebenenfalls wenigstens eine Gruppe umfasst, die ausgewählt ist unter S und einer Doppel- oder Dreifachbindung.

Eine weitere bevorzugte Ausführungsform sind Verbindungen der Formel IB, worin R¹, R² und R³ unabhängig voneinander für H, C₁-C₈-Alkyl, das gegebenenfalls durch OH, OC₁-C₈-Alkyl oder Halogen substituiert sein kann, OH, OC₁-C₈-Alkyl, SR⁴ oder NR⁴R⁵ steht, wobei R⁴ und R⁵ unabhängig voneinander für H oder C₁-C₈-Alkyl stehen;

Ar für Phenyl, Pyridyl oder Pyrimidyl steht, das gegebenenfalls einen, zwei, drei oder vier Substituenten aufweist, die ausgewählt sind unter C₁-C₈-Alkyl, das gegebenenfalls durch OH, OC₁-C₈-Alkyl oder Halogen substituiert ist, OR^{4a}, wobei R^{4a} für H, C₁-C₈-Alkyl, das gegebenenfalls durch OH, OC₁-C₈-Alkyl oder Halogen substituiert ist, steht, CHF₂, CF₃, CN, Halogen, C₂-C₆-Alkenyl, C₂-C₆-Alkinyl, C₃-C₆-Cyclohexyl, Phenyl, Naphthyl und einem 5- oder 6-gliedrigen heterocyclischen aromatischen Rest mit 1 bis 3 Heteroatomen, ausgewählt unter O, N und S.

Eine weitere bevorzugte Ausführungsform sind Verbindungen der Formel IB, worin B für 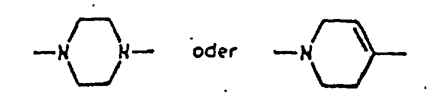 steht.

Eine weitere bevorzugte Ausführungsform sind Verbindungen der Formel IB, worin R¹ für H, C₁-C₈-Alkyl, das gegebenenfalls durch OH, OC₁-C₈-Alkyl oder Halogen substituiert ist, OR⁴, SR⁴ oder NR⁴R⁵ steht, wobei R⁴ und R⁵ unabhängig voneinander für H oder C₁-C₈-Alkyl stehen; R² für H, OR⁴ oder C₁-C₈-Alkyl steht; und R³ für H steht.

Eine weitere bevorzugte Ausführungsform sind Verbindungen der Formel IC, worin Ar für Phenyl steht, das gegebenenfalls ein bis vier Substituenten aufweist, die unabhängig voneinander ausgewählt sind unter C₁-C₈-Alkyl, das gegebenenfalls durch OH, OC₁-C₈-Alkyl oder Halogen substituiert ist, Phenyl, Naphthyl, pyrrolyl, CN, NO₂, CF₃, CHF₂, Halogen, SO₂R⁴ oder SR⁴ steht, wobei R⁴ für H oder C₁-C₈-Alkyl steht oder worin die Substituenten unabhängig voneinander ausgewählt sind unter C₁-C₈-Alkyl, Phenyl, CF₃, CHF₂, CN, NO₂, Halogen oder SR⁴ steht, wobei R⁴ für H oder C₁-C₈-Alkyl steht.

Eine weitere bevorzugte Ausführungsform ist die Verwendung von Verbindungen der Formel IA, worin Ar für Pyrimidinyl steht, das gegebenenfalls ein bis drei Substituenten aufweist, die unabhängig voneinander ausgewählt sind unter C₁-C₈-Alkyl, Phenyl, Naphthyl, C₅-C₆-Cycloalkyl, OH, OC₁-C₈-Alkyl, Halogen, CN, NO₂, CF₃, CHF₂ und einem 5- oder 6-gliedrigen, heterocyclischen, aromatischen oder nicht-aromatischen Rest mit 1 bis 3 Heteroatomen, ausgewählt unter O, N und S.

Eine weitere bevorzugte Ausführungsform ist die Verwendung von Verbindungen der Formel I', worin Ar für Pyridinyl steht, das gegebenenfalls ein bis vier Substituenten aufweist, die unabhängig voneinander ausgewählt sind unter C₁-C₈-Alkyl, Phenyl, Naphthyl, OH, OC₁-C₈-Alkyl, Halogen, CF₃, CN, C₂-C₆-Alkenyl, C₂-C₆-Alkinyl und einem 5-oder 6-gliedrigen, heterocyclischen, aromatischen Rest mit 1 bis 3 Heteroatomen, ausgewählt unter O, N und S.

Die Erfindung umfaßt auch die Säureadditionssalze der Verbindungen der Formel I, mit physiologisch verträglichen Säuren. Als physiologisch verträgliche organische und anorganische Säuren kommen beispielsweise Salzsäure, Bromwasserstoffsäure, Phosphorsäure, Schwefelsäure, Oxalsäure, Maleinsäure, Fumarsäure, Milchsäure, Weinsäure, Adipinsäure oder Benzoesäure in Betracht. Weitere brauchbare Säuren sind in Fortschritte der Arzneimittelforschung, Band 10, Seiten 224 ff, Birkhäuser Verlag, Basel und Stuttgart, 1966, beschrieben.

Die Verbindungen der Formel I können ein oder mehrere Asymmetriezentren aufweisen. Zur Erfindung zählen daher nicht nur die Racemate, sondern auch die betreffenden Enantiomere und Diastereomere. Auch die jeweiligen tautomeren Formen zählen zur Erfindung.

Die Herstellung der Verbindungen der Formel I' kann analog zu üblichen Methoden erfolgen, wie z.B. beschrieben in A.R. Katritzky, C.W. Rees (ed.), "Comprehensive Heterocyclic Chemistry", 1. Aufl. Pergamon Press 1984, insbesondere Vol. 3, Part 2A; D.J. Brown, "The Pyrimidines", in "The Chemistry of Heterocyclic Compounds", E.C. Taylor (Hrsg.), John Wiley & Sons Inc. NY, insbesondere Vol.16 + Suppl. I + II (1985), sowie Vol 52 (1994) und der dort zitierten Literatur. Das Verfahren zur Herstellung der Verbindungen besteht darin, daß man
i) eine Verbindung der allgemeinen Formel II: 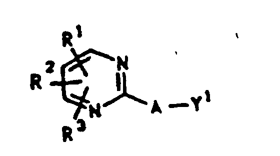 worin Y¹ für eine übliche Abgangsgruppe steht, mit einer Verbindung der allgemeinen Formel III

   H - B - Ar

   umsetzt;
ii) zur Herstellung einer Verbindung der Formel I',
   worin A ein Sauerstoff- oder Schwefelatom umfaßt:
   eine Verbindung der allgemeinen Formel IV: 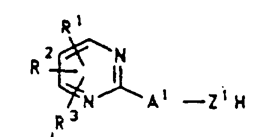
   worin Z¹ für O oder S steht und A¹ für C₀-C₁₄-Alkylen steht, mit einer Verbindung der allgemeinen Formel VI

   Y¹ - A² - B - Ar

   worin Y¹ die oben angegebenen Bedeutungen besitzt, und A² für C₁-C₁₅-Alkylen steht, wobei A¹ und A² zusammen 2 bis 15 Kohlenstoffatome aufweisen,
   umsetzt,
iii) zur Herstellung einer Verbindung der Formel I', worin A die Gruppe COO oder CONR⁴ umfaßt:
   eine Verbindung der allgemeinen Formel VII: 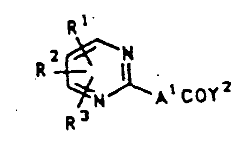 oder ein Salz davon, wobei Y² für OH, OC₁-C₄-Alkyl, Cl oder zusammen mit CO für eine aktivierte Estergruppe steht, und A¹ die oben angegebenen Bedeutungen besitzt, mit einer Verbindung der Formel VIII:

      Z¹ - A² - B - Ar
   worin A² die oben angegebenen Bedeutungen besitzt, und Z¹ für OH oder NHR⁴ steht, umsetzt,
iv) zur Herstellung einer Verbindung der Formel I', worin A die Gruppe OCO oder NR⁴CO umfaßt:
   eine Verbindung der Formel IV 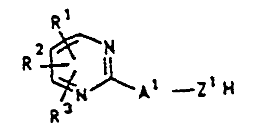
   worin Z¹ für O oder NR⁴ steht, mit einer Verbindung der Formel X:

   Y²CO - A² - B - Ar

   worin A², B und Y² die oben angegebenen Bedeutungen besitzen, umsetzt, wobei R¹, R², R³, A, B und Ar die oben angegebenen Bedeutungen besitzen.

Die oben beschriebenen Umsetzungen erfolgen im allgemeinen in einem Lösungsmittel bei Temperaturen zwischen Raumtemperatur und der Siedetemperatur des verwendeten Lösungsmittels. Brauchbare Lösungsmittel sind beispielsweise Ethylacetat, Tetrahydrofuran, Dimethylformamid, Dimethoxyethan, Toluol, Xylol oder ein Keton, wie Aceton oder Methylethylketon.

Gewünschtenfalls arbeitet man in Gegenwart eines säurebindenden Mittels. Geeignete säurebindende Mittel sind anorganische Basen, wie Natrium- oder Kaliumcarbonat, Natriummethylat, Natriumethylat, Natriumhydrid oder organische Basen, wie Triethylamin oder Pyridin. Letztere können gleichzeitig als Lösungsmittel dienen.

Die Isolierung des Rohprodukts erfolgt in üblicher Weise, beispielsweise durch Filtration, Abdestillieren des Lösungsmittels oder Extraktion aus dem Reaktionsgemisch etc. Die Reinigung der erhaltenen Verbindung kann in üblicher Weise erfolgen, beispielsweise durch Umkristallisieren aus einem Lösungsmittel, Chromatographie oder Überführen in eine Säureadditionsverbindung.

Die Säureadditionssalze werden in üblicher Weise durch Mischen der freien Base mit der entsprechenden Säure, gegebenenfalls in Lösung in einem organischen Lösungsmittel, beispielsweise einem niedrigen Alkohol, wie Methanol, Ethanol oder Propanol, einem Ether, wie Methyl-t-butylether, einem Keton, wie Aceton oder Methylethylketon oder einem Ester, wie Essigsäureethylester, hergestellt.

Die oben erwähnten Ausgangsmaterialien sind literaturbekannt oder können nach bekannten Verfahren hergestellt werden.

Zur Behandlung der oben erwähnten Erkrankungen werden die erfindungsgemäßen Verbindungen in üblicher Weise oral oder parenteral (subkutan, intravenös, intramuskulär, intraperitoneal) verabreicht. Die Applikation kann auch mit Dämpfen oder Sprays durch den Nasen-Rachen-Raum erfolgen.

Die Dosierung hängt vom Alter, Zustand und Gewicht des Patienten sowie von der Applikationsart ab. In der Regel beträgt die tägliche Wirkstoffdosis etwa 10 bis 1000 mg pro Patient und Tag bei oraler Gabe und etwa 1 bis 500 mg pro Patient und Tag bei parenteraler Gabe.

Die Erfindung betrifft auch pharmazeutische Mittel, die die erfindungsgemäßen Verbindungen IA, IB, IC oder ID enthalten. Diese Mittel liegen in den üblichen galenischen Applikationsformen in fester oder flüssiger Form vor, beispielsweise als Tabletten, Filmtabletten, Kapseln, Pulver, Granulate, Dragees, Suppositorien, Lösungen oder Sprays. Die Wirkstoffe können dabei mit den üblichen galenischen Hilfsmitteln, wie Tablettenbindemitteln, Füllstoffen, Konservierungsmitteln, Tablettensprengmitteln, Fließregulierungsmitteln, Weichmachern, Netzmitteln, Dispergiermitteln, Emulgatoren, Lösungsmitteln, Retardierungsmitteln, Antioxidantien und/oder Treibgasen verarbeitet werden (vgl. H. Sucker et al., Pharmazeutische Technologie, Thieme-Verlag, Stuttgart, 1978). Die so erhaltenen Applikationsformen enthalten den Wirkstoff normalerweise in einer Menge von 1 bis 99 Gew.-%.

Die nachfolgenden Beispiele dienen zur Erläuterung der Erfindung ohne sie zu begrenzen.

### Beispiel 1

### 4-[3-(4-{3-Trifluormethylphenyl}piperazinyl)-propylthio]-pyrimidin

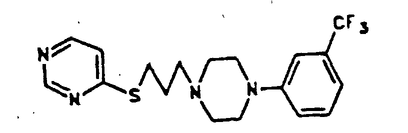

a) 1-(3-Chlorphenyl)-4-(3-trifluormethylphenyl)piperazin
   30 g (0,13 mol) m-Trifluormethylphenylpiperazin, 23 g (0,146 mol) 1-Brom-3-chlorpropan und 15 g (0,148 mol) Triethylamin wurden in 200 ml THF 4 Stunden unter Rückfluß erhitzt. Nach Abkühlen wurde abgesaugt und eingeengt. Der zähflüssige Rückstand wurde mit Essigester aufgenommen, mit Wasser gewaschen, über MgSO₄ getrocknet und anschließend eingeengt. Als Rückstand erhielt man 39 g Produkt als gelbliches Öl (quantitative Ausbeute).
b) 4-[3-(4-{3-Trifluormethylphenyl}piperazinyl)-propylthio]-pyrimidin
   1,5 g (13,4 mmol) 4-Mercaptopyrimidin, 4,3 g (14 mmol) 1-(3-Chlorpropyl)-4-(3-trifluormethylphenyl)piperazin und 1,5 g (15 mmol) Triethylamin wurden in 5 ml DMF 1 Stunde bei 100°C gerührt. Anschließend wurde auf 5%ige Salzsäure gegossen und nit MTB-Ether extrahiert. Nach Alkalisieren der wäßrigen Phase mit Natronlauge wurde mit Essigester extrahiert, die organische Phase über MgSO₄ getrocknet und eingeengt. Der Rückstand wurde chromatographisch gereinigt (Laufmittel : CH₂Cl₂/CH₃OH = 98/2). Es wurden 3,0 g Produkt als gelbliches Öl erhalten ( = 59 % Ausbeute)

| | | | | |
|---|---|---|---|---|
| H-NMR [δ,ppm] | 1,95(2H); | 2,55(2H); | 2,65 (4H); | 3,25(6H); |
| | 7,06(3H); | 7,15(1H); | 7,35(1H), | 8,33(1H); |
| | 8,9(1H) | | | |

### Beispiel 2

### 2-(5-(4-{3-Trifluormethylphenyl}piperazinyl)-pentylmercapto]-pyrimidin

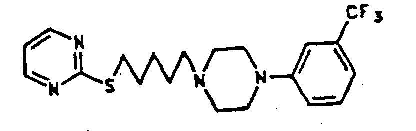

a) 2-(5-Chlorpentylmercapto)-pyrimidin
   2,8 g (25 mmol) 2-Mercaptopyrimidin, 4,64 g (25 mmol) 1-Brom-5-chlorpentan und 2,58 g (25,5 mmol) Triethylamin wurden in 100 ml THF 4 Stunden unter Rückfluß erhitzt. Nach dem Abkühlen wurde abgesaugt, eingeengt und der Rückstand chromatographisch gereinigt (Laufmittel : Cyclohexan/Essigester = 92/8). Man erhielt 2,8 g Produkt (= 52 % Ausbeute).
b) 2-[5-(4-{3-Trifluormethylphenyl}piperazinyl)-pentylmercapto]-pyrimidin
   2,8 g (12,9 mmol) 2-(5-Chlorpentylmercapto)-pyrimidin, 3,27 g (14,2 mmol) m-Trifluormethylphenylpiperazin und 1,44 g (14,2 mmol) Triethylamin wurden in 5 ml DMF 1 Stunde bei 90°C gerührt. Anschließend wurde auf Wasser gegossen und dreimal mit CH₂Cl₂ extrahiert, über MgSO₄ getrocknet und eingeengt. Der Rückstand wurde mit Methyl-t-butylether versetzt, abgesaugt und die Mutterlauge eingeengt. Nach chromatographischer Reinigung (Laufmittel : CH₂Cl₂/CH₃OH = 97/3) erhielt man 4,0 g Produkt als Öl ( = 75 % Ausbeute)
¹H-NMR [δ;ppm]: 1,54(4H); 1,78 (2H); 2,4 (2H); 2,6 (4H); 3,18
(2H); 3,23 (4H); 6,95 (1H); 7,01(3H); 7,1 (3H); 7,33 (1H); 8,5
(1H).

In analoger Weise wurden die in der nachfolgenden Tabelle 1 angegebenen Verbindungen hergestellt:

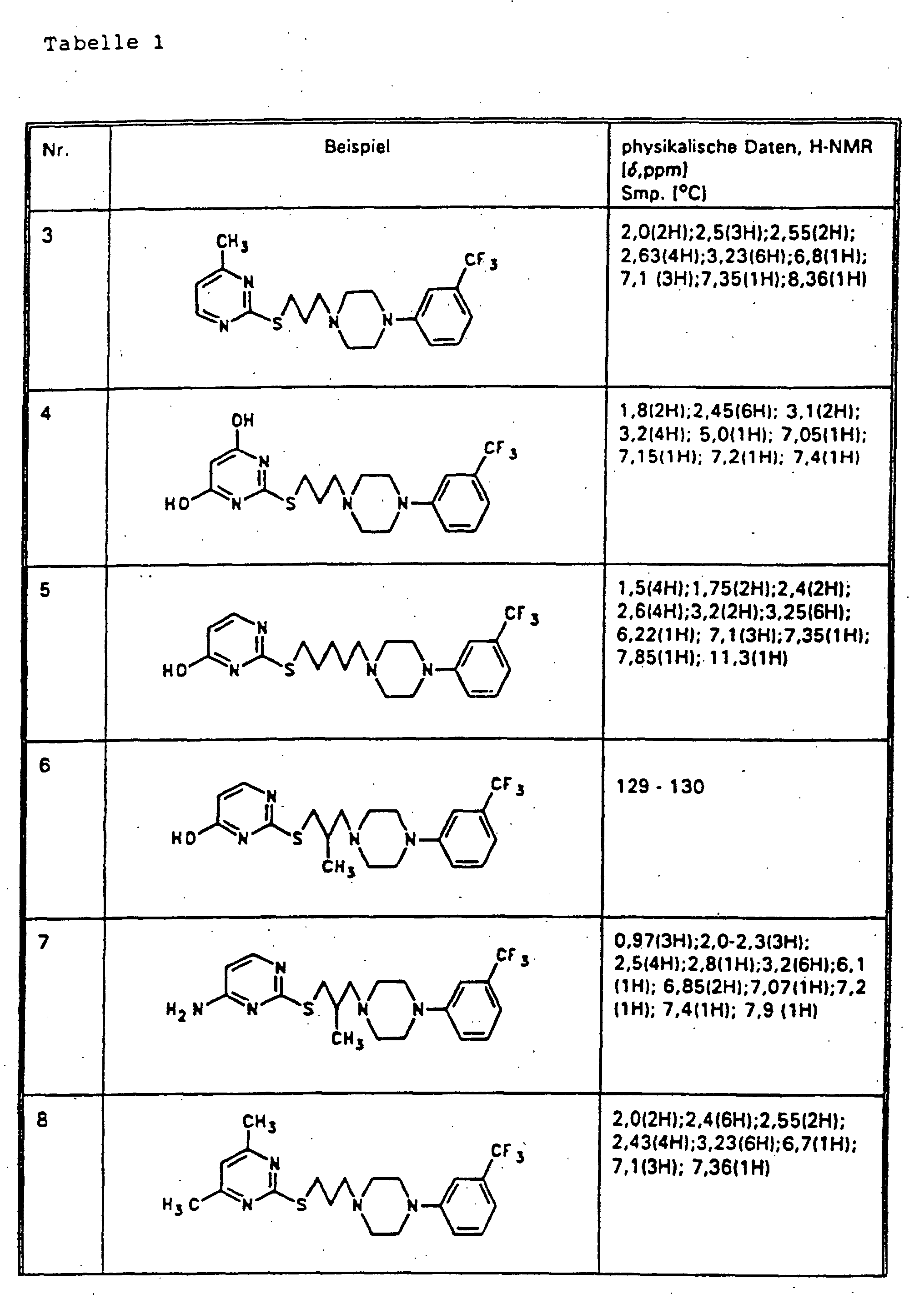

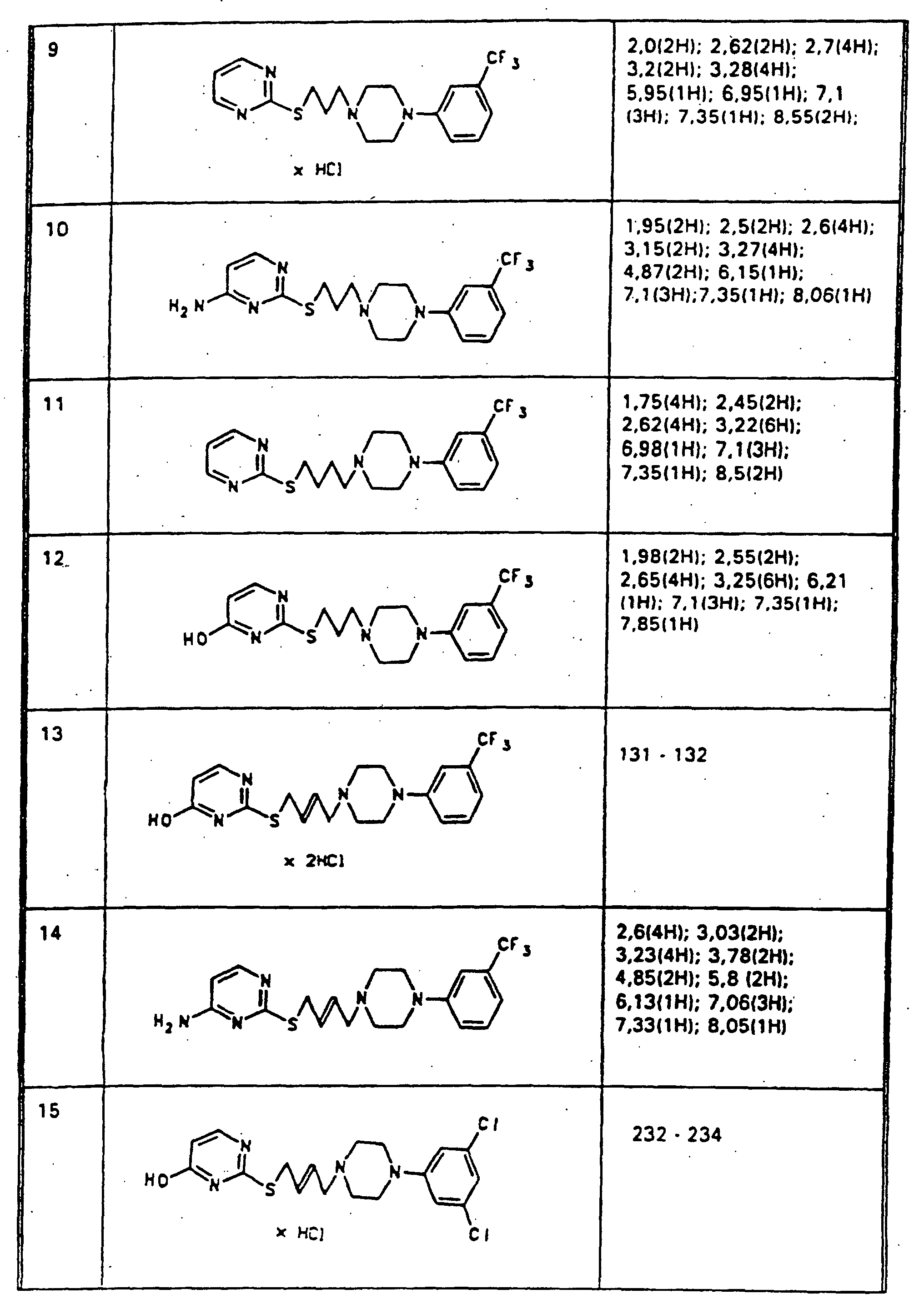

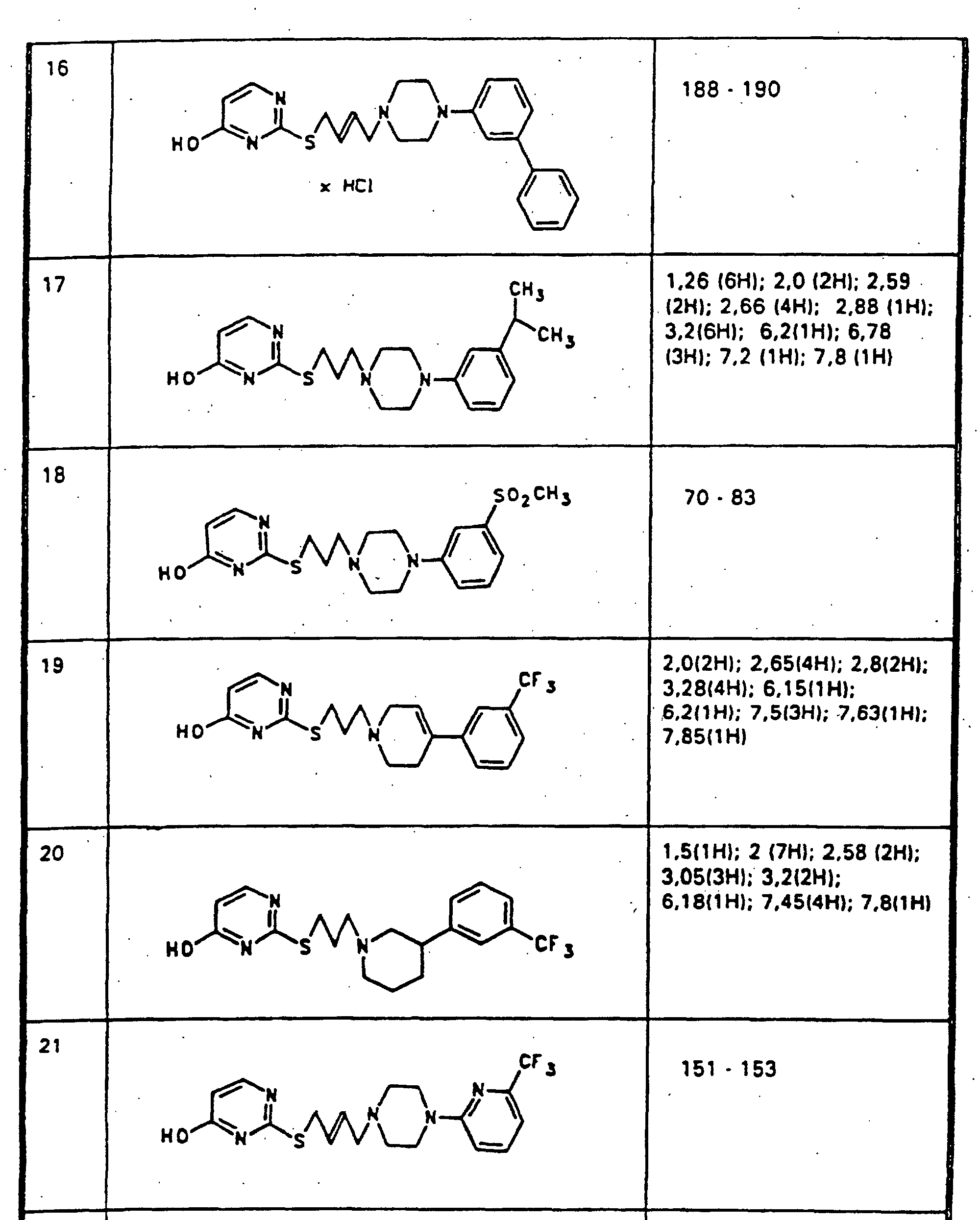

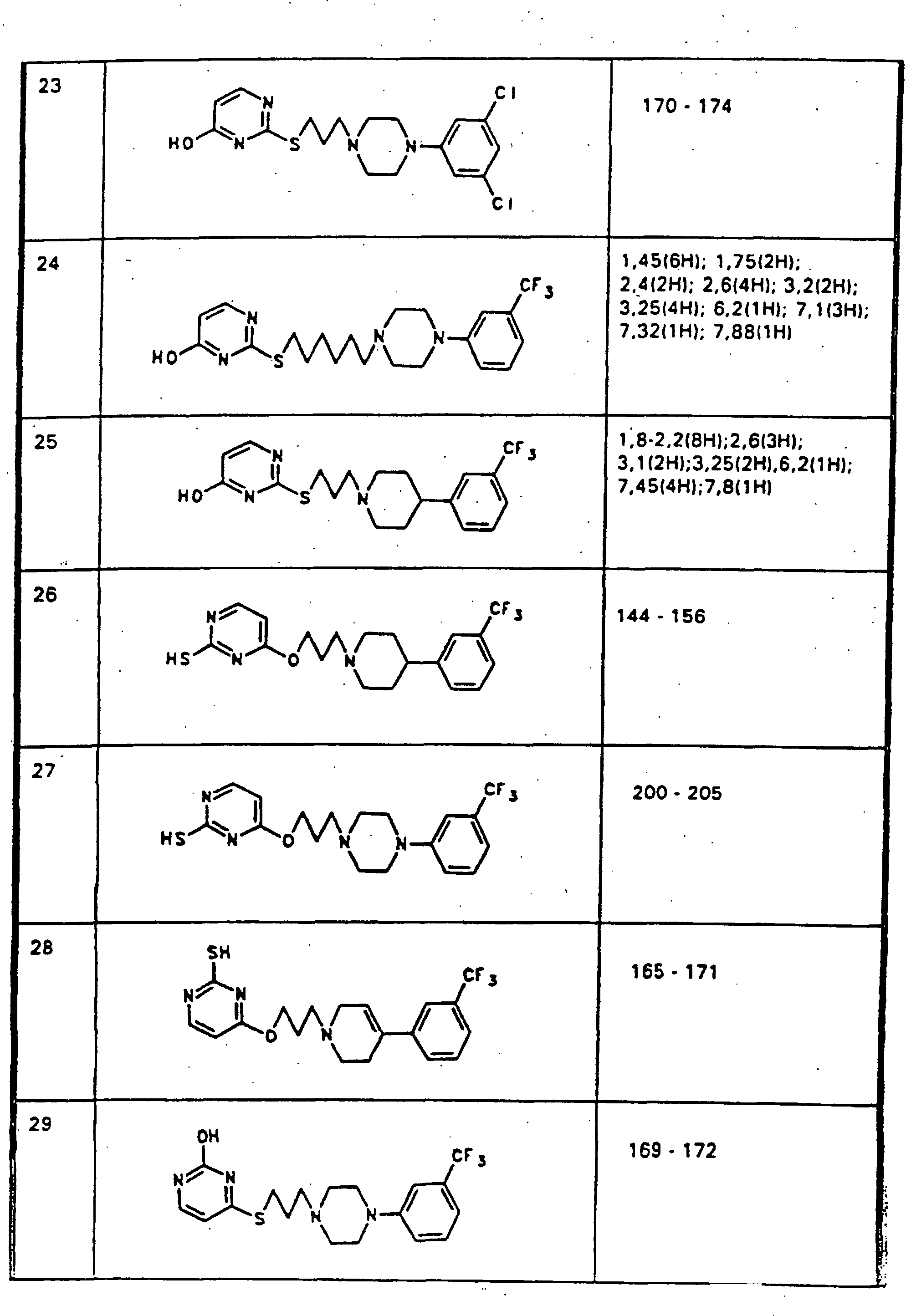

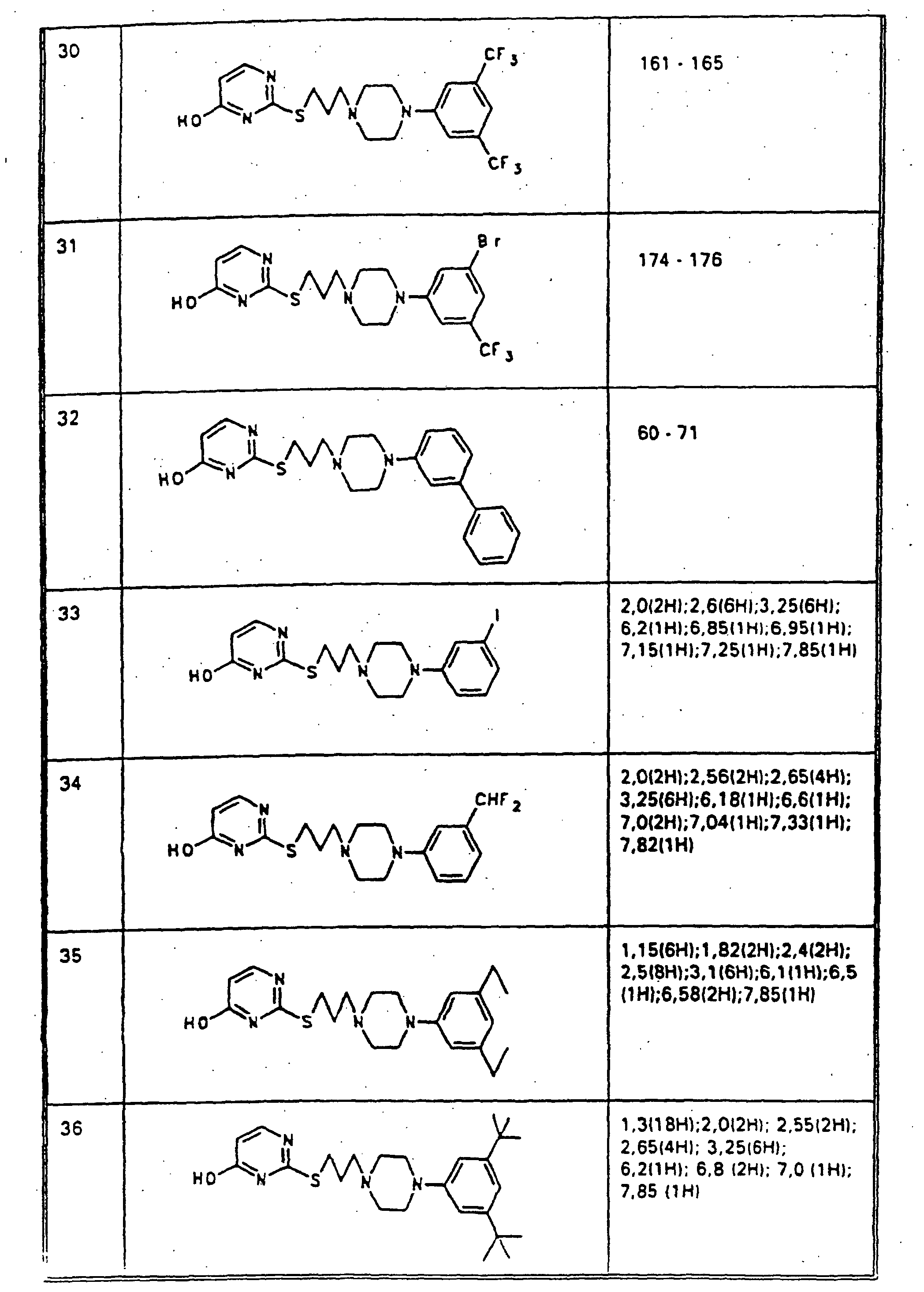

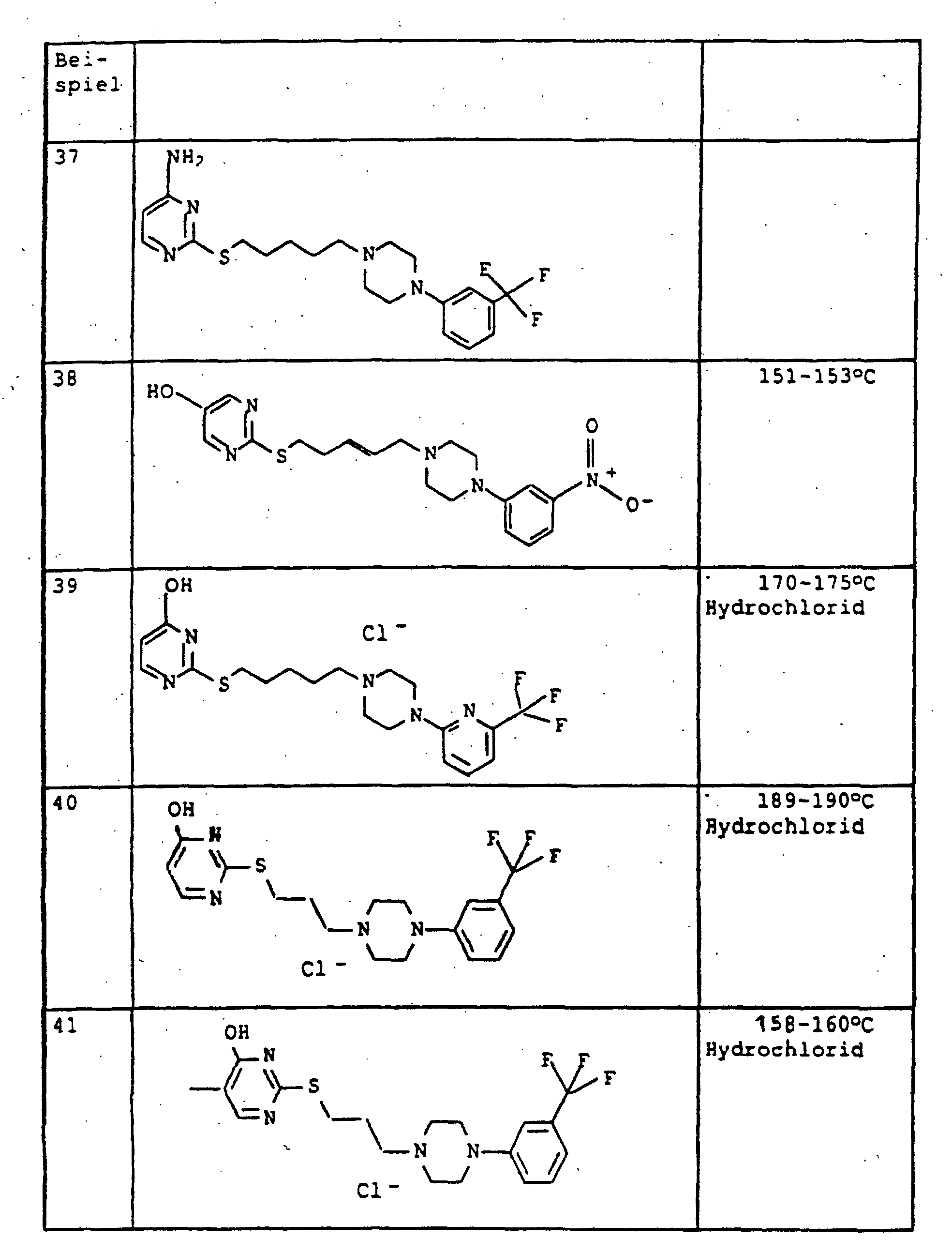

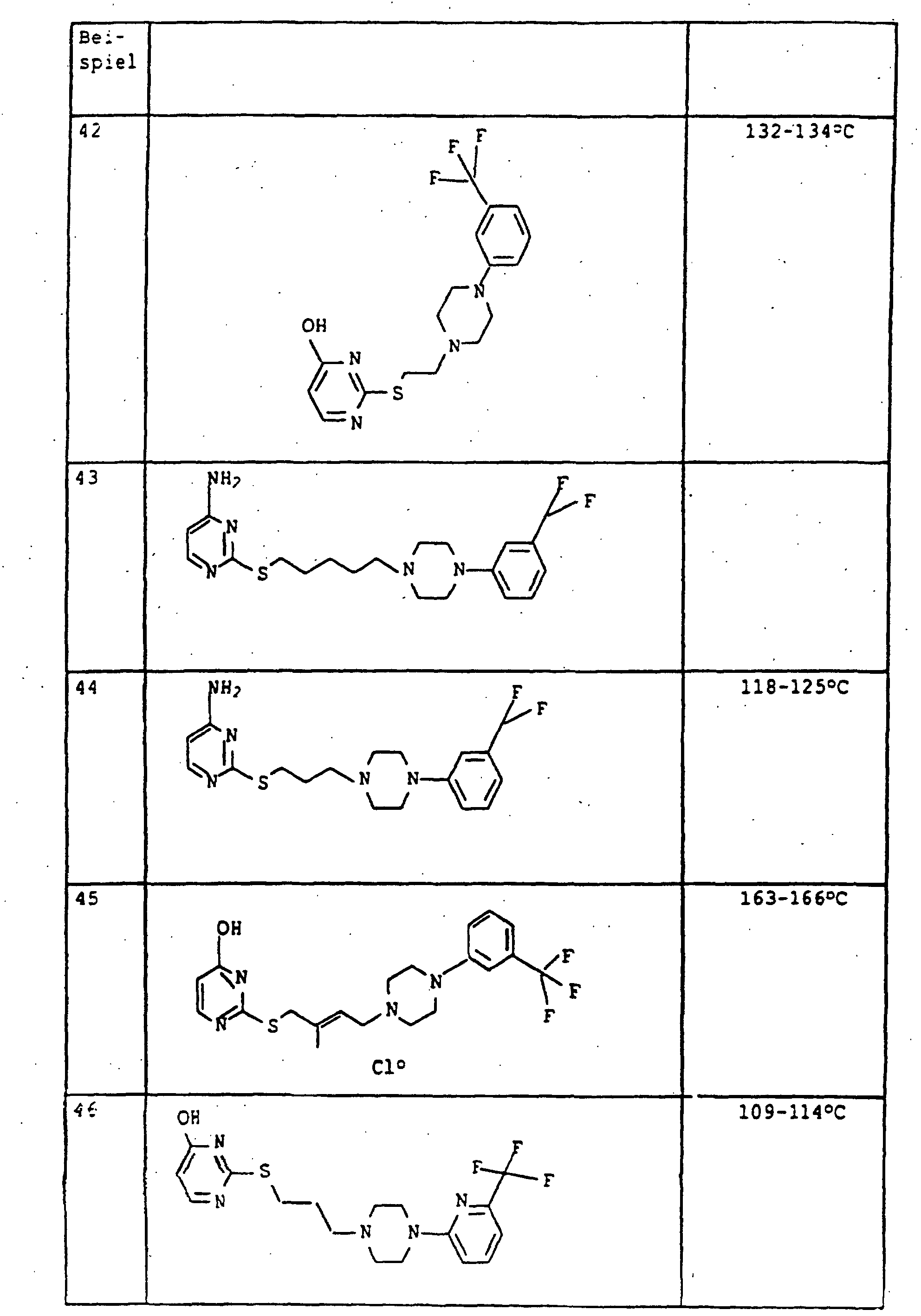

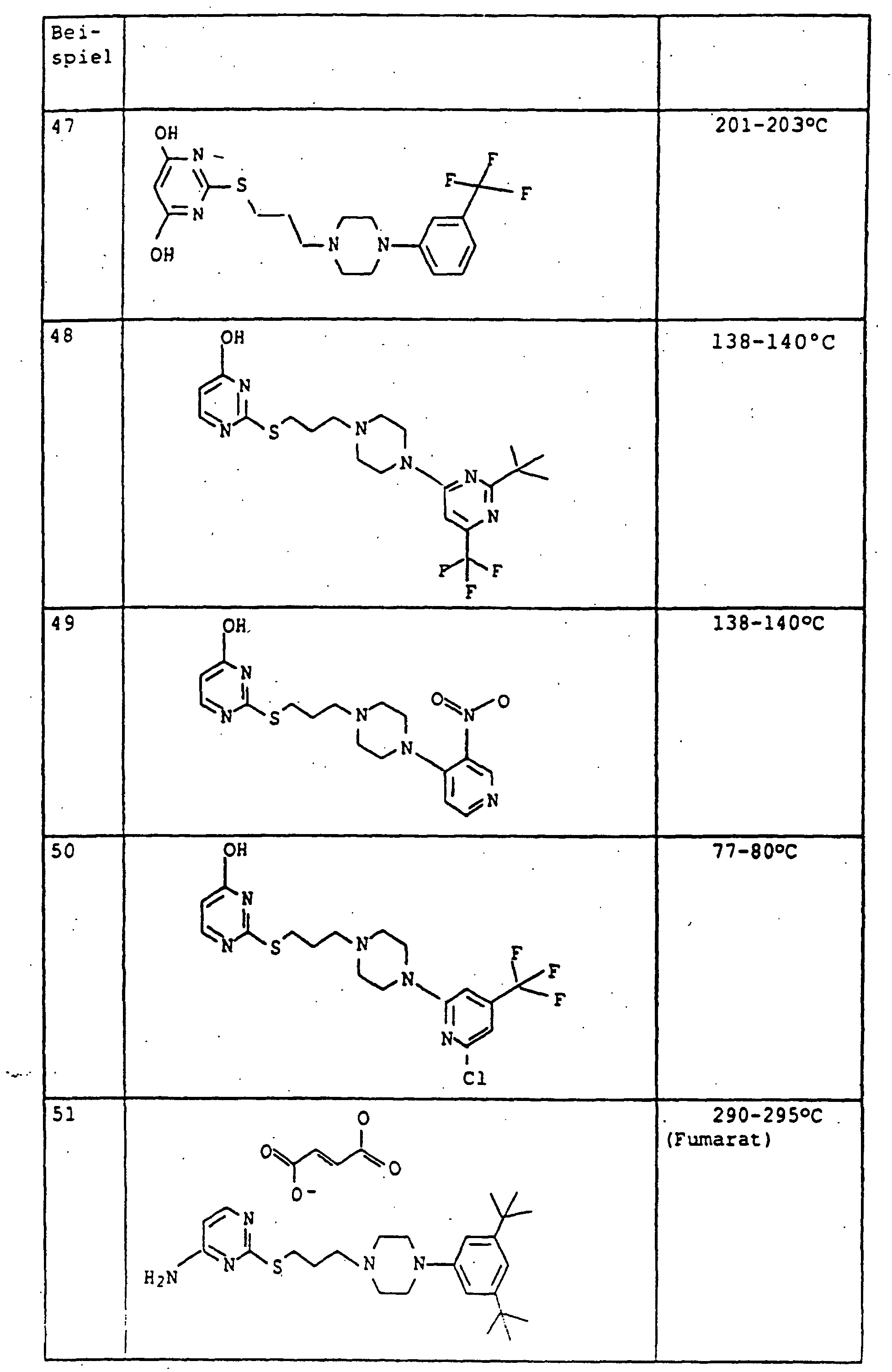

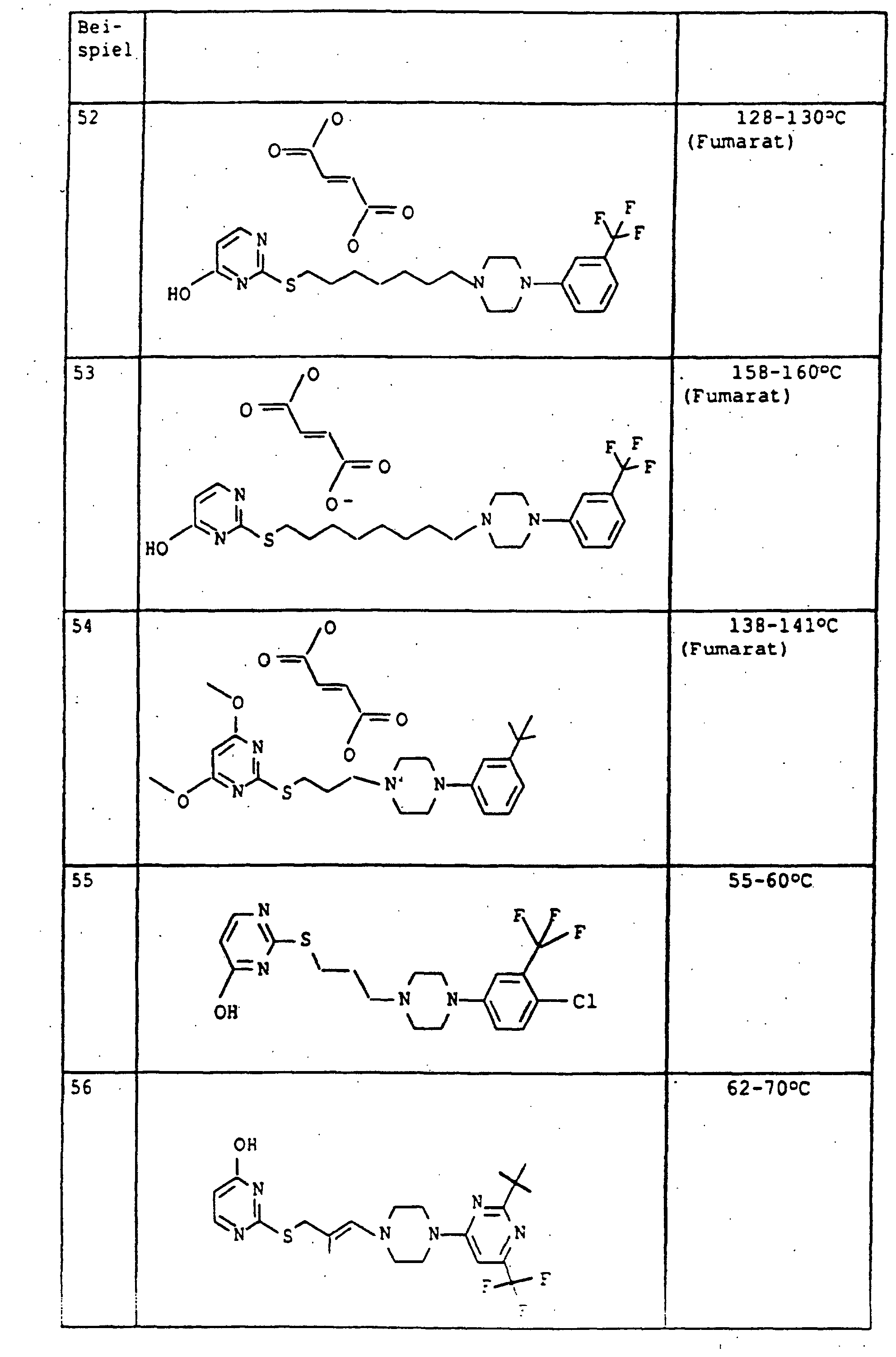

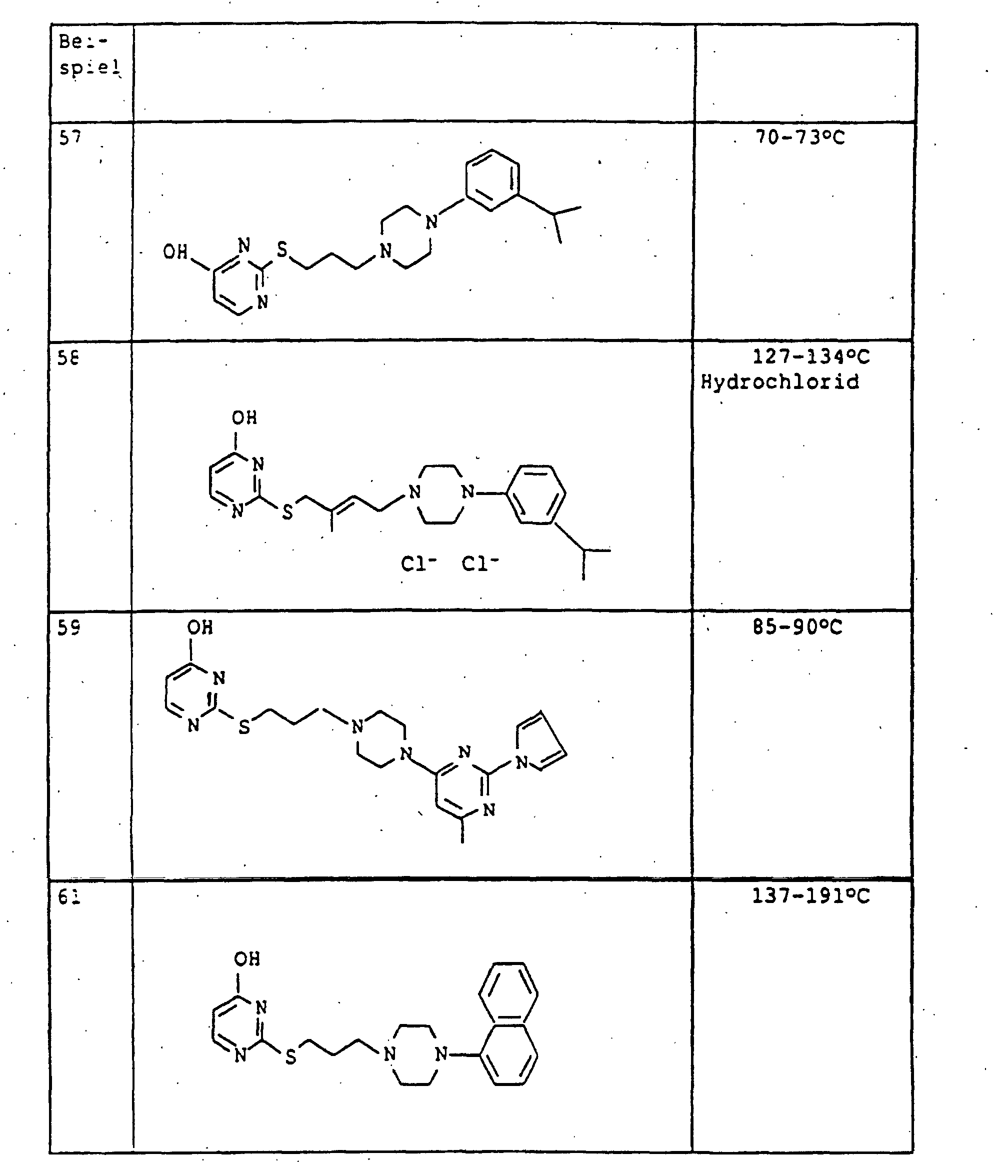

Auf analoge Weise können die in den nachfolgenden Tabellen 2-6 genannten Verbindungen erhalten werden.

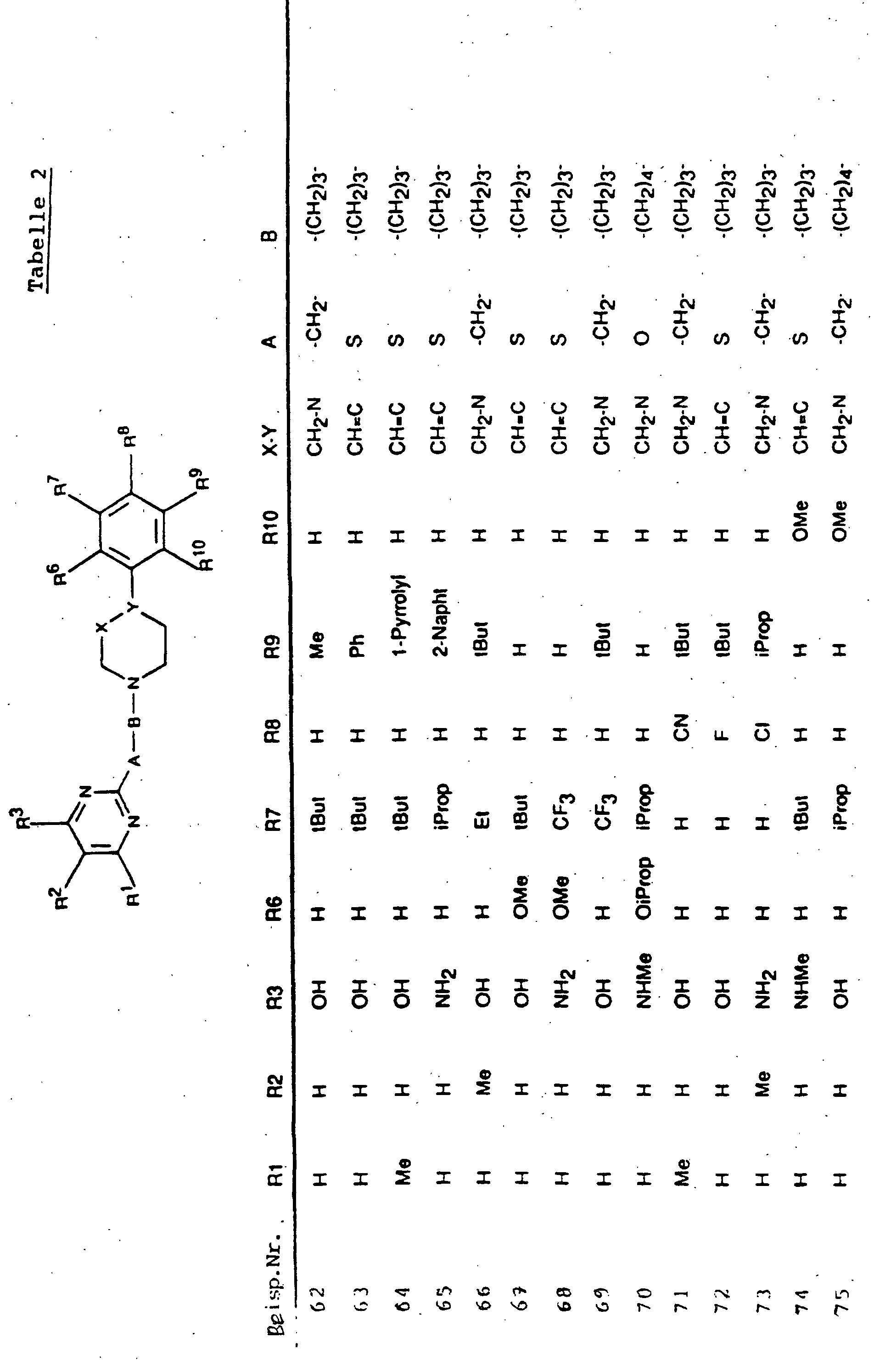

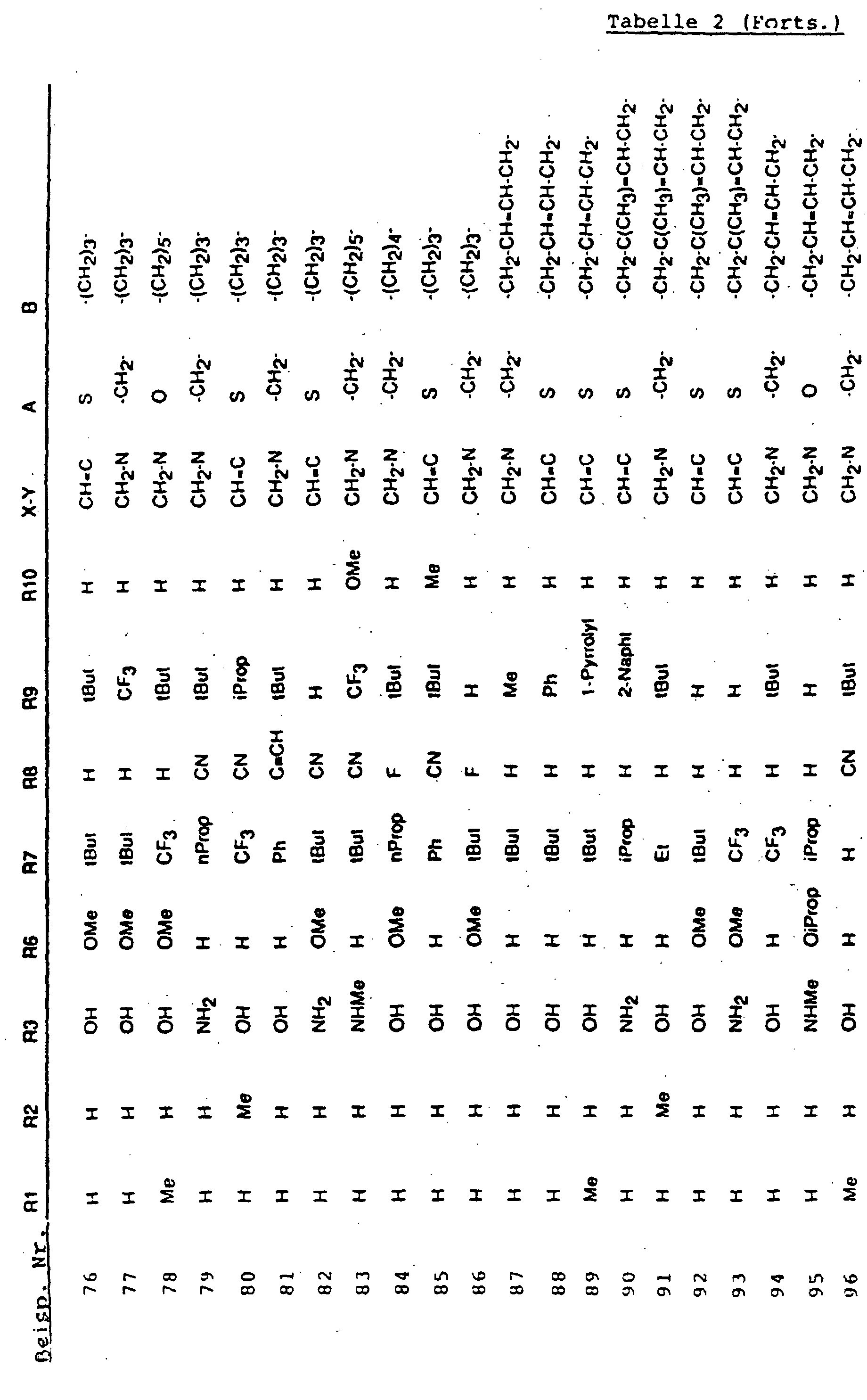

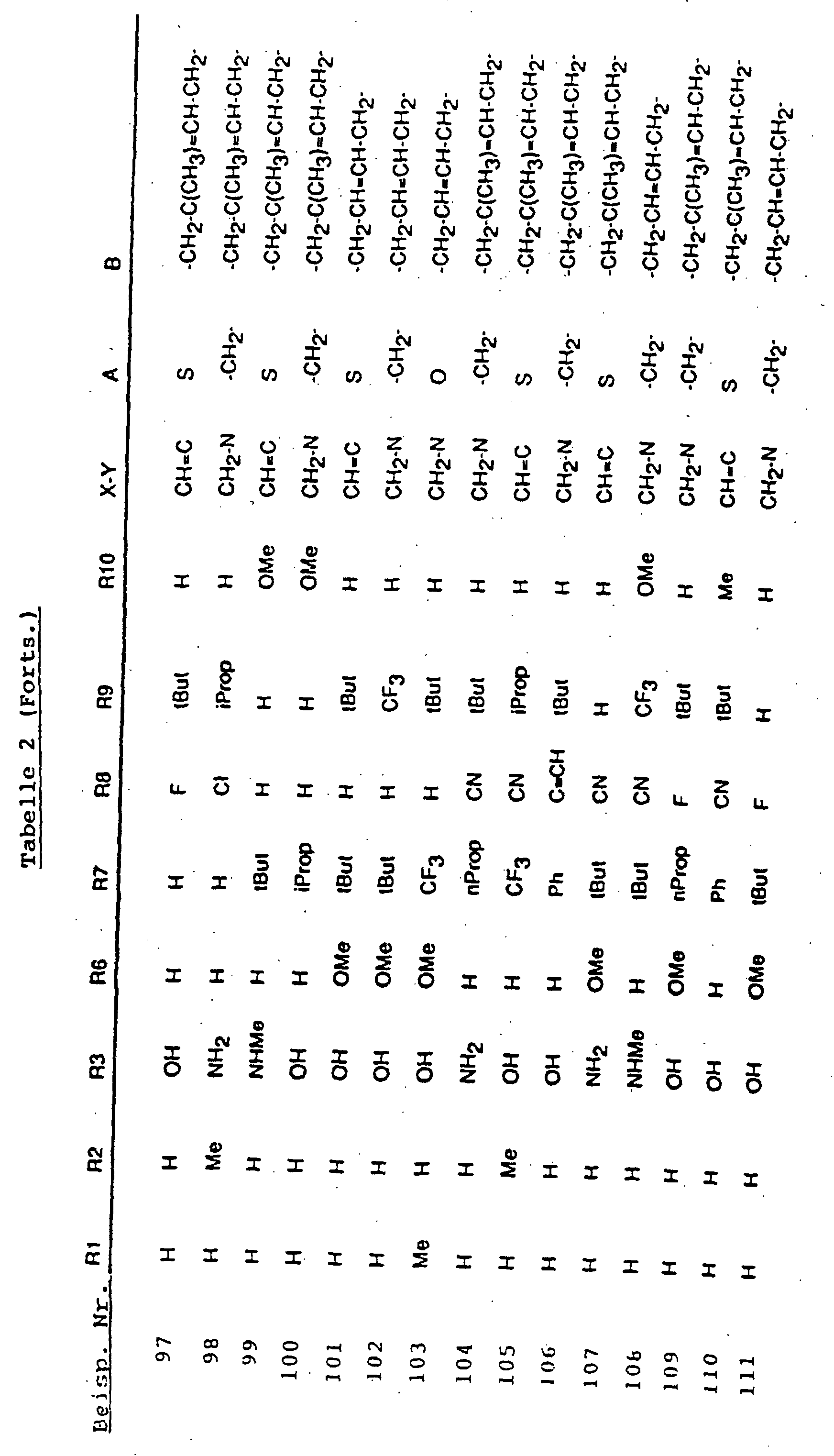

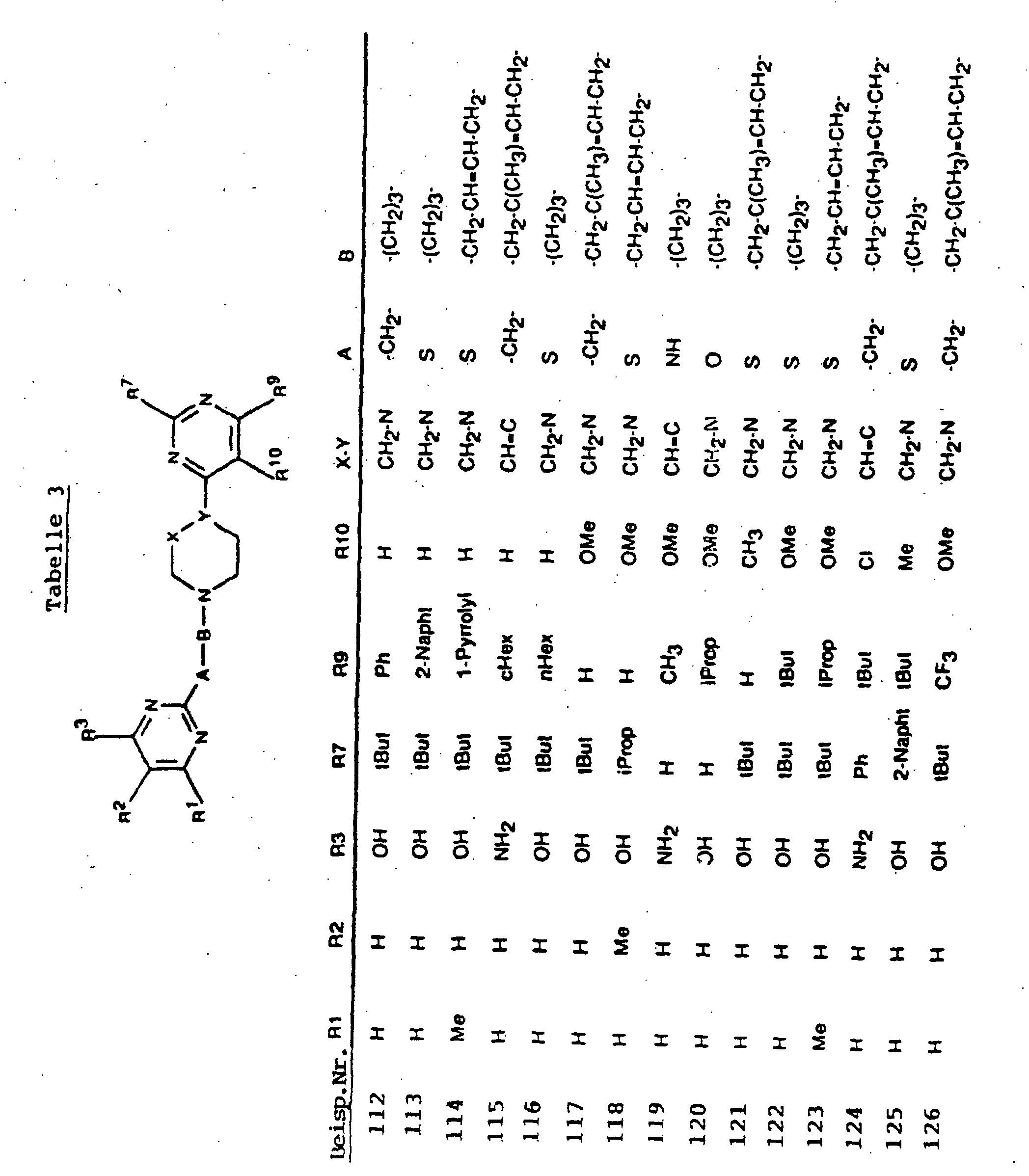

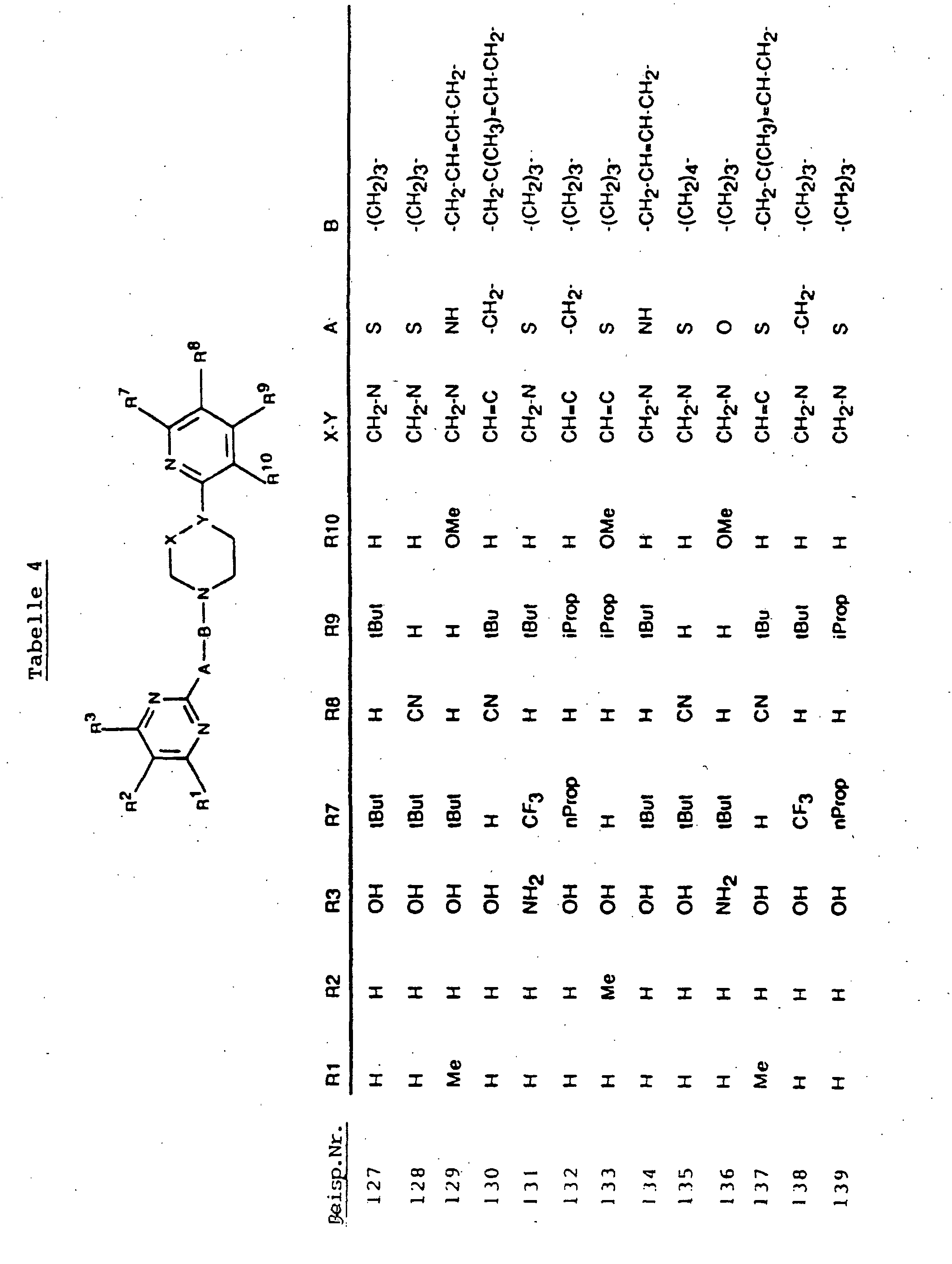

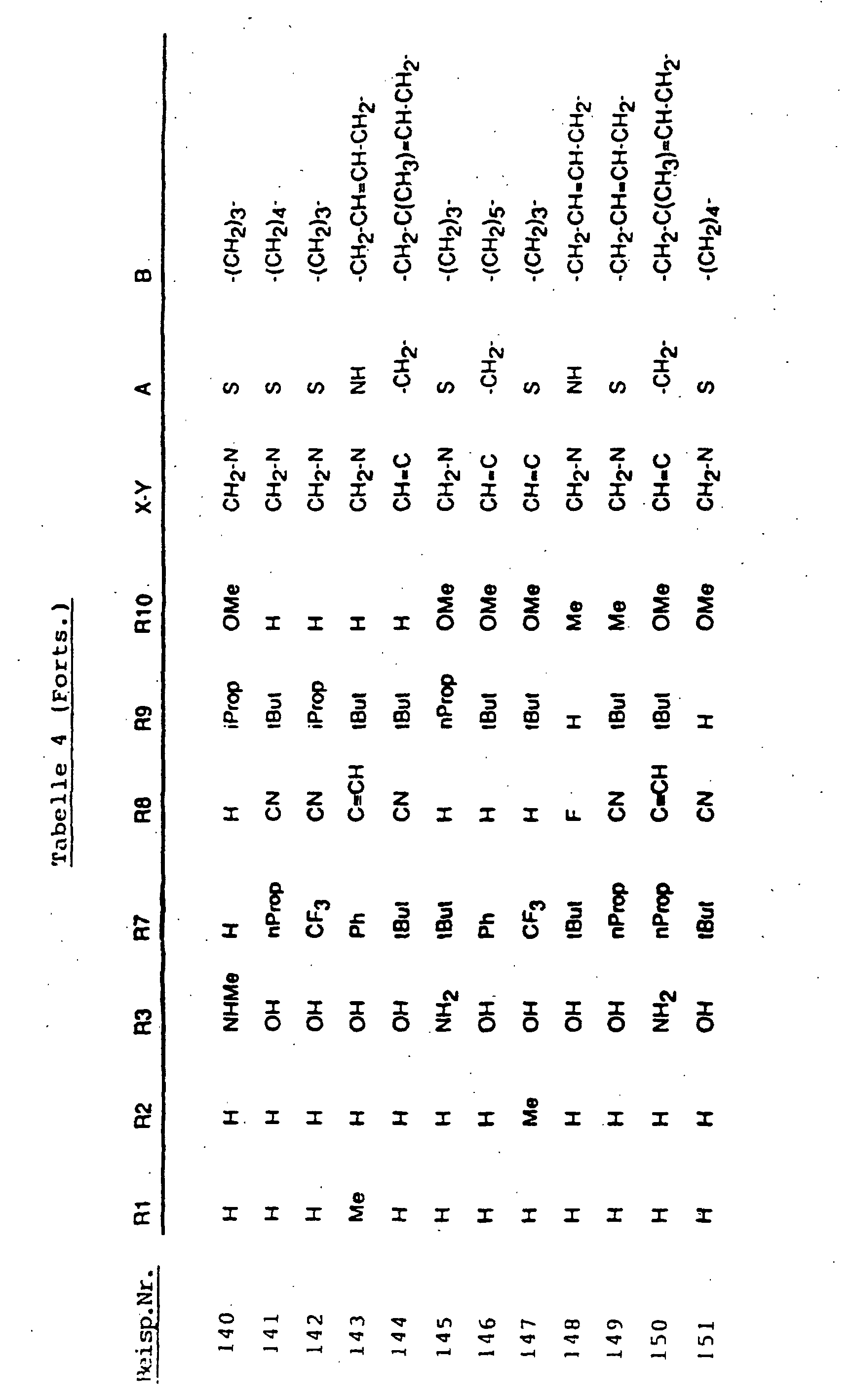

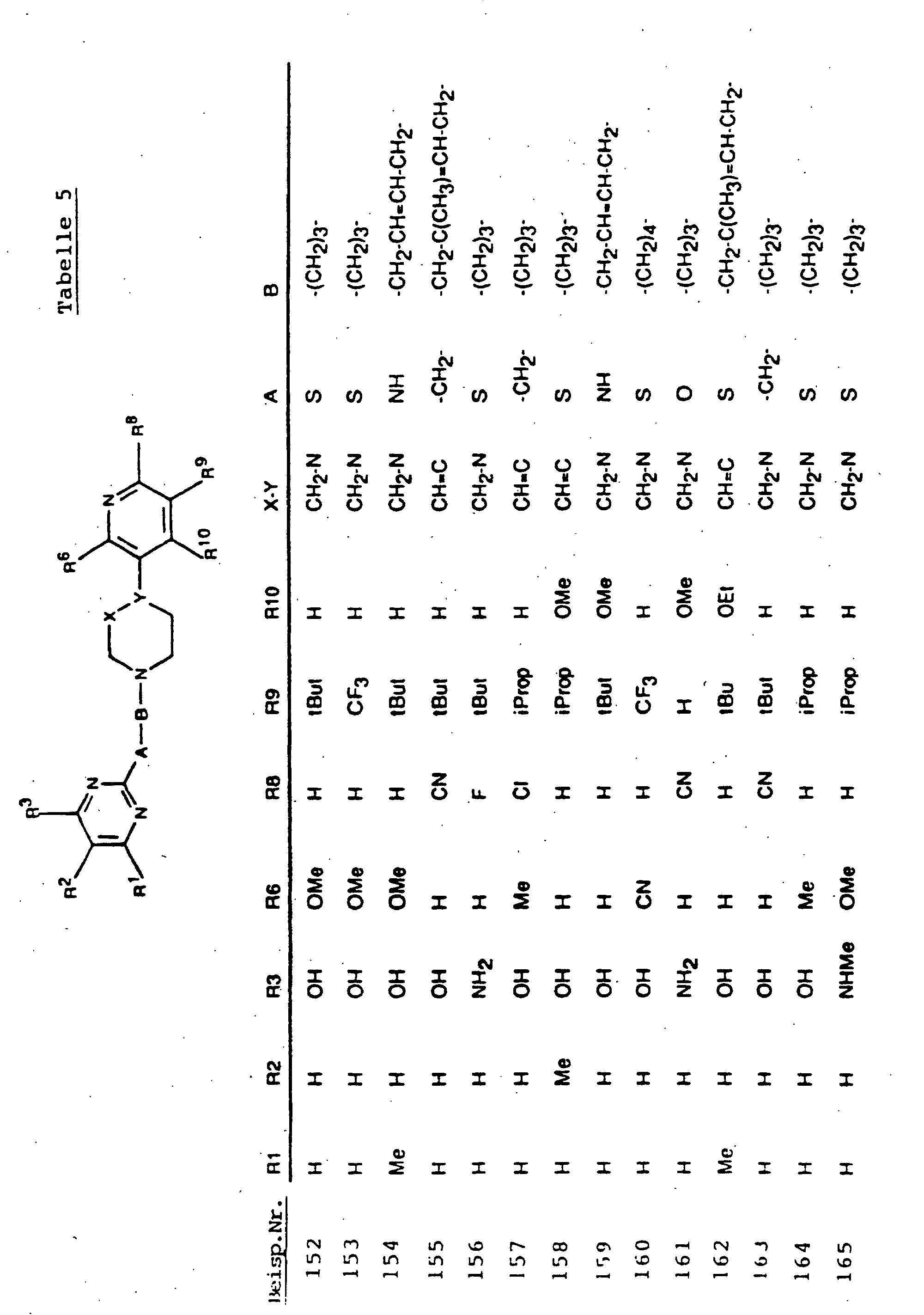

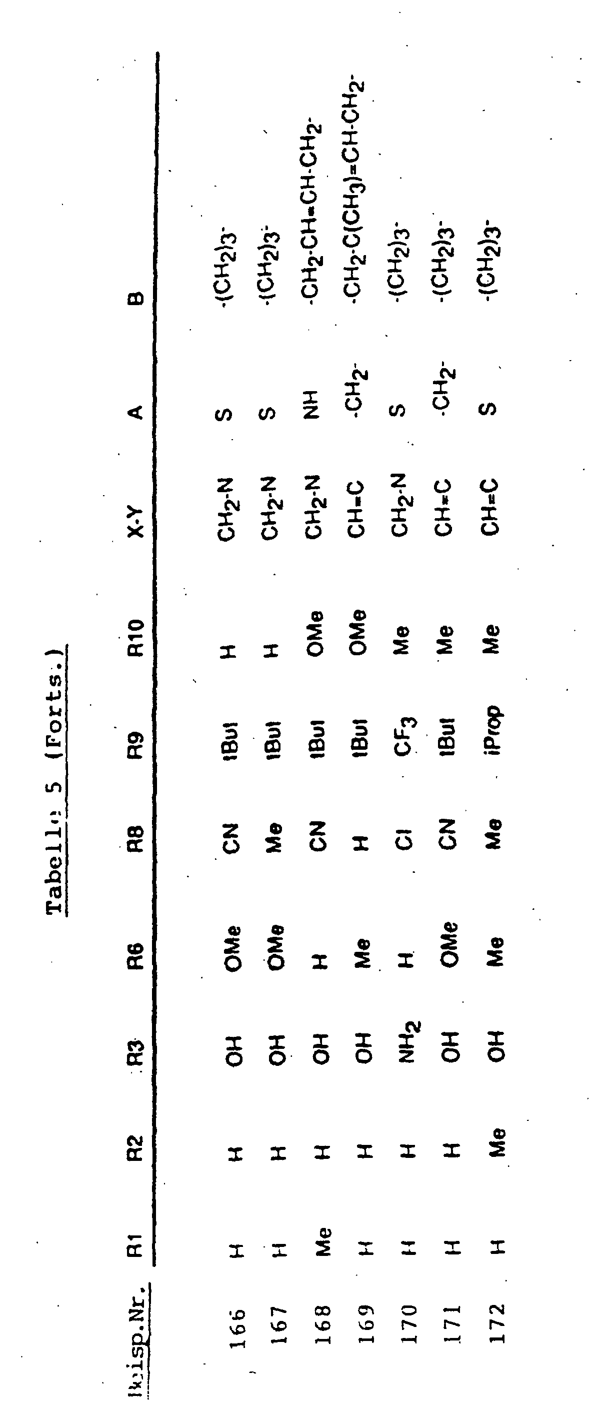

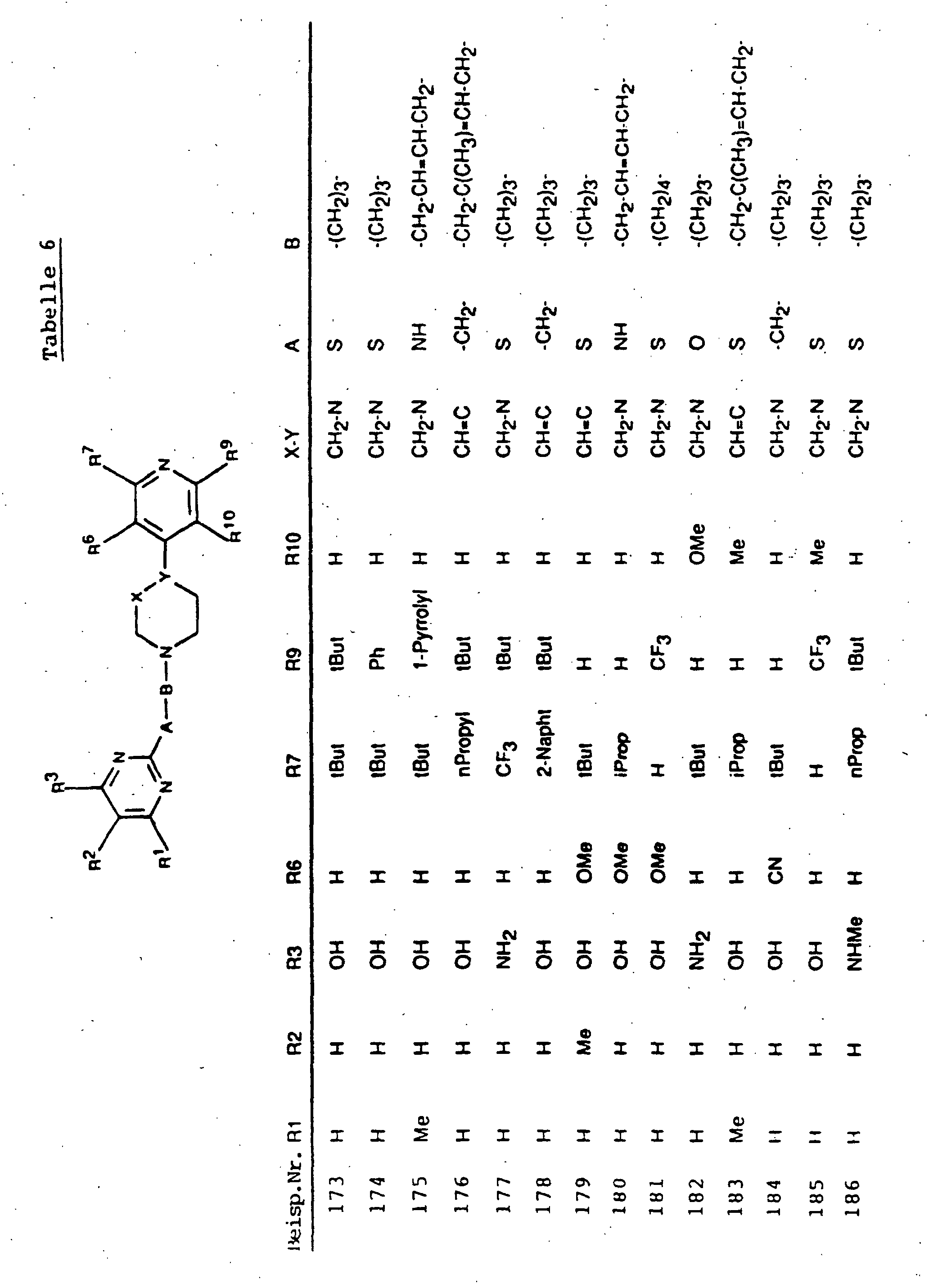

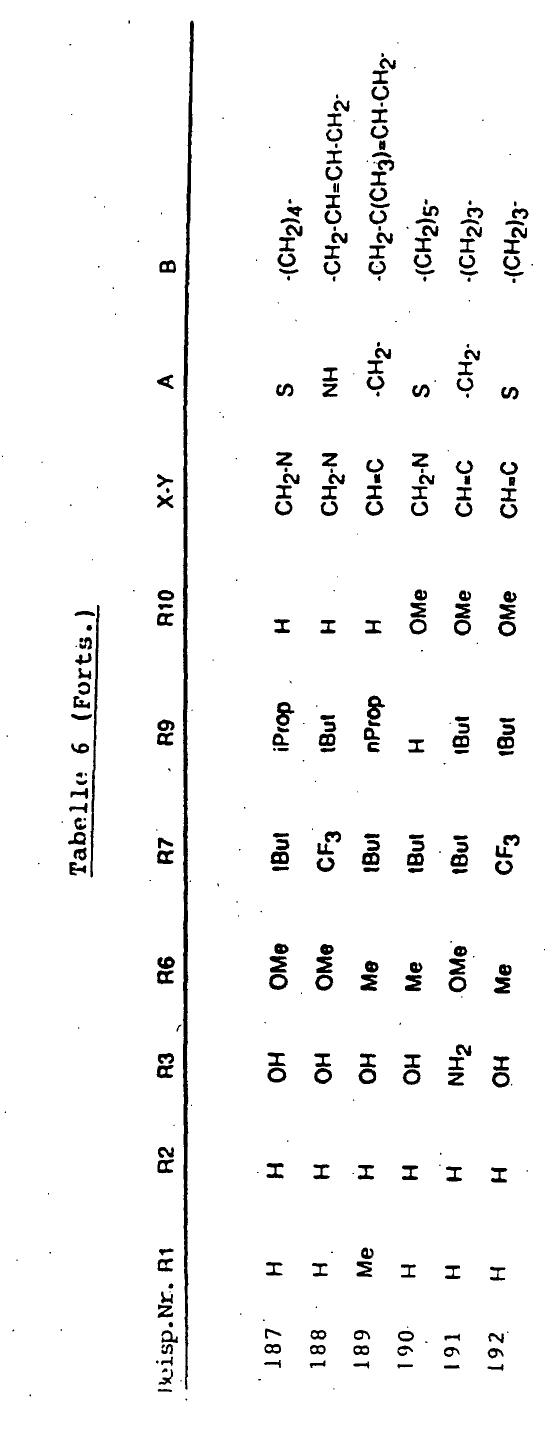

### Beispiele für galenische Applikationsformen:

### A) Tabletten

Auf einer Tablettenpresse werden in üblicher Weise Tabletten folgender Zusammensetzung gepreßt:

| | |
|---|---|
| 40 mg | Substanz des Beispiels 1 |
| 120 mg | Maisstärke |
| 13,5 mg | Gelatine |
| 45 mg | Milchzucker |
| 2,25 mg | Aerosil® (chemisch reine Kieselsäure in submikroskopisch feiner Verteilung) |
| 6,75 mg | Kartoffelstärke (als 6 %iger Kleister) |

### B) Dragees

| | |
|---|---|
| 20 mg | Substanz des Beispiels 4 |
| 60 mg | Kernmasse |
| 70 mg | Verzuckerungsmasse |

Die Kernmasse besteht aus 9 Teilen Maisstärke, 3 Teilen Milchzucker und 1 Teil Vinylpyrrolidon-Vinylacetat-Mischpolymerisat 60:40. Die Verzuckerungsmasse besteht aus 5 Teilen Rohrzucker, 2 Teilen Maisstärke, 2 Teilen Calciumcarbonat und 1 Teil Talk. Die so hergestellten Dragees werden anschließend mit einem magensaftresistenten Überzug versehen.

### Biologische Untersuchungen - Rezeptorbindungsstudien

### 1) D₃-Bindungstest

Für die Bindungsstudien wurden klonierte humane D₃-Rezeptor-exprimierende CCL 1,3 Mäusefibroblasten, erhältlich bei Res. Biochemicals Internat. One Strathmore Rd., Natick, MA 01760-2418 USA, eingesetzt.

### Zellpräparation

Die D₃ exprimierenden Zellen wurden in RPMI-1640 mit 10 % fötalem Kälberserum (GIBCO Nr. 041-32400 N); 100 E/ml Penicillin und 0,2 % Streptomycin (GIBCO BRL, Gaithersburg, MD, USA) vermehrt. Nach 48 h wurden die Zellen mit PBS gewaschen und mit 0,05 % trypsinhaltiger PBS 5 min inkubiert. Danach wurde mit Medium neutralisiert und die Zellen durch Zentrifugation bei 300 xg gesammelt. Zur Lyse der Zellen wurde kurz das Pellet mit Lysispuffer (5mM Tris-HCl, pH 7,4 mit 10 % Glycerin) gewaschen und danach in einer Konzentration von 10⁷-Zellen /ml Lysispuffer 30 min bei 4°C inkubiert. Die Zellen wurden bei 200 xg 10 min zentrifugiert und das Pellet in flüssigem Stickstoff gelagert.

### Bindungstests

Für den D₃-Rezeptorbindungstest wurden die Membranen in Inkubationspuffer (50 mM Tris-HCl, pH 7,4 mit 120 mM NaCl, 5 mM KCl, 2 mM CaCl₂, 2 mM MgCl₂, 10µM Quinolinol, 0,1 % Ascorbinsäure und 0,1 % BSA) in einer Konzentration von ca. 10⁶ Zellen/250 µl Testansatz suspendiert und bei 30°C mit 0,1 nM ¹²⁵Jodsulpirid in Anwesenheit und Abwesenheit von Testsubstanz inkubiert. Die unspezifische Bindung wurde mit 10⁻⁶M Spiperon bestimmt.

Nach 60 min wurde der freie und der gebundene Radioligand durch Filtration über GF/B Glasfaserfilter (Whatman, England) an einem Skatron-Zellsammler (Skatron, Lier, Norwegen) getrennt und die Filter mit eiskaltem Tris-HCl-Puffer, pH 7,4 gewaschen. Die auf den Filtern gesammelte Radioaktivität wurde mit einem Packard 2200 CA Flüssigkeitszintillationszähler quantifiziert.

Die Bestimmung der Kᵢ-Werte erfolgte über nichtlineare Regressionsanalyse mit dem Programm LIGAND.

### 2) D₂-Bindungstest

### Membranpräparationen

### a) Nucleus caudatus (Rind)

Nucleus caudatus wurde aus Rinderhirn entfernt und in eiskalter 0,32 M Saccharose-Lösung gewaschen. Nach Gewichtsbestimmung wurde das Material zerkleinert und in 5 - 10 Volumen Saccharose-Lösung mit einem Potter-Elvehjem Homogenisator (500 U/min) homogenisiert. Das Homogenat wurde bei 3000 x g 15 Minuten (4°C) zentrifugiert und der resultierende Überstand einer weiteren 15minütigen Zentrifugation bei 40000 x g unterworfen. Danach wurde der Rückstand zweimal mit 50 mM Tris-HCl, pH 7,4 durch Resuspension und Zentrifugation gewaschen. Die Membranen wurden bis zum Gebrauch in flüssigem N₂ gelagert.

### b) Striatum (Ratte)

Striati von Sprague-Dawley Ratten wurden in eiskalter 0,32 M Saccharose-Lösung gewaschen. Nach Gewichtsbestimmung wurden die Hirnteile in 5 - 10 Volumen Saccharose-Lösung mit einem Potter-Elvehjem Homogenisator (500 U/min) homogenisiert. Das Homogenat wurde bei 40000 x g 10 Minuten (4°C) zentrifugiert, danach wurde der Rückstand mit 50 mM Tris-HCl, 0.1 mM EDTA und 0,01 % Ascorbinsäure (pH 7,4) mehrmals durch Resuspension und Zentrifugation gewaschen. Der gewaschene Rückstand wurde mit dem obengenannten Puffer resuspendiert und 20 Minuten bei 37°C inkubiert (zwecks Abbau des endogenen Dopamins). Anschließend wurden die Membranen zweimal mit Puffer gewaschen und in Portionen in flüssigem Stickstoff eingefroren.Die Membranpräparation war maximal 1 Woche stabil.

### Bindungstest

a) ³H-Spiperon (D_{2low})
   Nucleus caudatus-Membranen wurden in Inkubationspuffer (mM: Tris-HCl 50, NaCl 120, KC1 5, MgCl₂ 1, CaCl₂ 2, pH 7,4) aufgenommen. Verschiedene Ansätze von je 1 ml wurden hergestellt:
   - Totale Bindung: 400 µg Membranen + 0,2 nmol/l ³H-Spiperon (Du Pont de Nemours, NET-565).
   - Unspezifische Bindung: wie Ansätze für totale Bindung + 10 µM (+)-Butaclamol.
   - Prüfsubstanz: wie Ansätze für totale Bindung + steigende Konzentrationen von Prüfsubstanz.

   Nach erfolgter Inkubation bei 25°C für 60 Minuten wurden die Ansätze über GF/B Glasfaserfilter (Whatman, England) an einem Skatron-Zellsammler (Fa. Zinsser, Frankfurt) filtriert und die Filter mit eiskaltem 50 mM Tris-HCl-Puffer, pH 7,4 gewaschen. Die auf den Filtern gesammelte Radioaktivität wurde mit einem Packard 2200 CA Flüssigkeitszintillationszähler quantifiziert.
   Die Bestimmung der Kᵢ-Werte erfolgte über nichtlineare Regressionsanalyse mit dem Programm LIGAND oder durch Umrechnung der IC₅₀-werte mit Hilfe der Formel von Cheng und Prusoff.
b) ³H-ADTN (D_{2high})
   Striatummembranen wurden in Inkubationspuffer (50 mM Tris-HCl, pH 7,4, 1 mM MnCl₂ und 0,1 % Ascorbinsäure) aufgenommen.
   Verschiedene Ansätze von je 1 ml wurden hergestellt.
   - Totale Bindung: 300 µg Naßgewicht + 1 nM ³H-ADTN (Du Pont de Nemours, Kundensynthese) + 100 nM SCH 23390 (Belegung von D1-Rezeptoren).
   - Unspezifische Bindung: wie Ansätze für totale Bindung + 50 nM Spiperon.
   - Prüfsubstanz: wie Ansätze für totale Bindung + steigende Konzentrationen von Prüfsubstanz.

   Nach erfolgter Inkubation bei 25°C für 60 Minuten wurden die Ansätze über GF/B Glasfaserfilter (Whatman, England) an einem Skatron-Zellsammler (Fa. Zinsser, Frankfurt) filtriert und die Filter mit eiskaltem 50 mM Tris-HCl-Puffer, pH 7,4 gewaschen. Die auf den Filtern gesammelte Radioaktivität wurde mit einem Packard 2200 CA Flüssigkeitszintillationszähler quantifiziert.
   Die Auswertung erfolgte wie unter a).

Die erfindungsgemäßen Verbindungen zeigen in diesen Tests sehr gute Affinitäten und hohe Selektivitäten gegenüber dem D₃-Rezeptor. Die erhaltenen Werte sind für repräsentative Verbindungen in der nachfolgenden Tabelle 7 zusammengestellt.

**Tabelle 7**

| Rezeptorbindung | | | |
|---|---|---|---|
| Beispiel-Nr. | D₃ ¹²⁵I-Sulpirid Kᵢ [nM] | D₂ ³H-Spiperon Kᵢ [mM] | Selektivität Kᵢ D₂/Kᵢ.D₃ |
| 12 | 4,2 | 357 | 85 |
| 13 | 2,3 | 142 | 61 |
| 17 | 2,8 | 200 | 71 |
| 19 | 3,0 | 175 | 58 |
| 48 | 4,0 | 480 | 120 |

## Patentansprüche

1. Pyrimidinverbindungen der Formel IA: 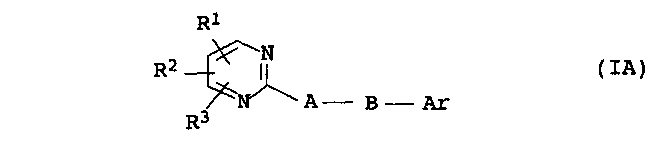 worin
A für eine C₂-C₁₅-Alkylengruppe steht, die gegebenenfalls mindestens eine Gruppe umfaßt, die ausgewählt ist unter S, NR⁴, CONR⁴, NR⁴CO, COO, OCO und einer Doppel- oder einer Dreifachbindung,
B für 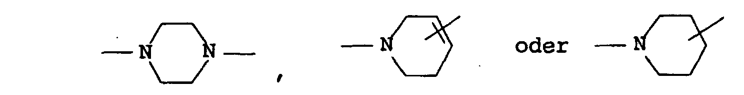 steht,
R¹, R², R³ unabhängig voneinander ausgewählt sind unter H, Halogen, OR⁴, NR⁴R⁵, SR⁴, CF₃, CN, CO₂R⁴ und C₁-C₈-Alkyl, das gegebenenfalls durch OH, OC₁-C₈-Alkyl oder Halogen substituiert ist,
R⁴ für H, C₁-C₈-Alkyl, das gegebenenfalls durch OH, OC₁-C₈-Alkyl oder Halogen substituiert ist, steht,
R⁵ die für R⁴ angegebenen Bedeutungen besitzt oder für COR⁴ oder CO₂R⁴ steht;
Ar für Phenyl, Pyridyl, Pyrimidyl oder Triazinyl steht,
wobei Ar gegebenenfalls ein bis vier Substituenten aufweisen kann, die unabhängig voneinander ausgewählt sind unter OR⁵, C₁-C₈-Alkyl, C₂-C₆-Alkenyl, C₂-C₆-Alkinyl, Halogen, CN, CO₂R⁴, NO₂, SO₂R⁴, SO₃R⁴, NR⁴R⁵, SO₂NR⁴R⁵, SR⁴, CF₃, CHF₂, einem 5- oder 6-gliedrigen carbocyclischen, aromatischen oder nicht-aromatischen Ring und einem 5-oder 6-gliedrigen heterocyclischen aromatischen oder nichtaromatischen Ring mit 1 bis 3 Heteroatomen, die ausgewählt sind unter O, S und N, wobei der Ring gegebenenfalls substituiert sein kann durch C₁-C₈-Alkyl, Hal, OC₁-C₈-Alkyl, OH, NO₂, CF₃ und wobei
Ar gegebenenfalls auch mit einem 5- oder 6-gliedrigen carbocyclischen, aromatischen oder nicht-aromatischen Ring oder einem 5- oder 6-gliedrigen, aromatischen oder nicht-aromatischen, heterocyclischen Ring mit 1 bis 3 Heteroatomen, die ausgewählt sind unter O, S und N, kondensiert sein kann,
wobei Ar nicht für Phenyl steht, wenn A eine Gruppe NR⁴ umfasst,
und die Salze davon mit physiologisch verträglichen Säuren,
ausgenommen die Verbindungen der Formel 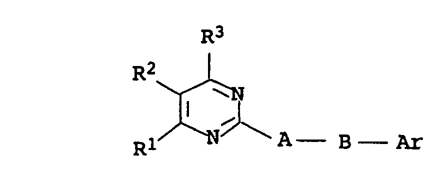 worin R¹ für OH oder SH steht, R² und R³ unabhängig voneinander für H, C₁-C₆-Alkyl, OC₁-C₆-Alkyl, SC₁-C₆-Alkyl, CO₂H, OH, SH, NR⁴R⁵ oder Halogen stehen, wobei R⁴ und R⁵ für H oder C₁-C₆-Alkyl stehen, A für SC₁-C₆-Alkylen,
B für 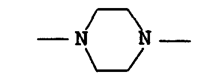 steht und Ar für Phenyl steht, das gegebenenfalls einen oder mehrere Substituenten aufweist, die ausgewählt sind unter C₁-C₄-Alkyl, OC₁-C₄-Alkyl, SC₁-C₄-Alkyl, NO₂, CF₃, F, Cl oder Br.

2. Pyrimidinverbindungen der Formel IA nach Anspruch 1, wobei
Ar für Phenyl, Pyridyl, Pyrimidyl oder Triazinyl steht, wobei Ar gegebenenfalls einen oder zwei Substituenten X und Y aufweisen kann, die unabhängig voneinander ausgewählt sind unter OR⁵, C₁-C₈-Alkyl, Hal, CN, CO₂R⁴, NO₂, SO₂R⁴, SO₃R⁴, NR⁴R⁵, SO₂NR⁴R⁵, SR⁴, CF₃, CHF₂, einem 5- oder 6-gliedrigen carbocyclischen, aromatischen oder nicht-aromatischen Ring und einem 5- oder 6-gliedrigen heterocyclischen aromatischen oder nicht-aromatischen Ring mit 1 bis 3 Heteroatomen, die ausgewählt sind unter O, S und N, wobei der Ring gegebenenfalls substituiert sein kann durch C₁-C₈-Alkyl, Hal, OC₁-C₈-Alkyl, OH, NO₂, CF₃ und wobei
Ar gegebenenfalls auch mit einem 5- oder 6-gliedrigen carbocyclischen, aromatischen oder nicht-aromatischen Ring oder einem 5- oder 6-gliedrigen, aromatischen oder nicht-aromatischen oder heterocyclischen Ring mit 1 bis 3 Heteroatomen, die ausgewählt sind unter O, S und N, kondensiert sein kann.

3. Verbindungen der Formel IA nach Anspruch 1 oder 2, wobei A für C₃-C₁₀-Alkylen steht, das gegebenenfalls wenigstens eine Gruppe umfaßt, die ausgewählt ist unter S und einer Doppeloder Dreifachverbindung.

4. Verbindungen der Formel IB 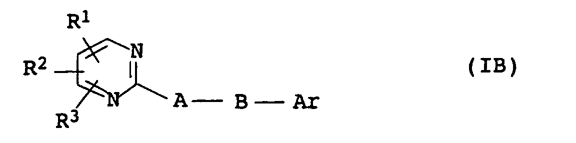 worin R¹, R² und R³ unabhängig voneinander für H, C₁-C₈-Alkyl, das gegebenenfalls durch OH, OC₁-C₈-Alkyl oder Halogen substituiert sein kann, OH, OC₁-C₈-Alkyl, SR⁴ oder NR⁴R⁵ steht, wobei R⁴ und R⁵ unabhängig voneinander für H oder C₁-C₈-Alkyl stehen;
Ar für Phenyl, Pyridyl oder Pyrimidyl steht, das gegebenenfalls einen, zwei, drei oder vier Substituenten aufweist, die ausgewählt sind unter C₁-C₈-Alkyl, das gegebenenfalls durch OH, OC₁-C₈-Alkyl oder Halogen substituiert ist, OR^{4a}, wobei R^{4a} für H, C₁-C₈-Alkyl, das gegebenenfalls durch OH, OC₁-C₈-Alkyl oder Halogen substituiert ist, steht, CHF₂, CF₃, CN, Halogen, C₂-C₆-Alkenyl, C₂-C₆-Alkinyl, C₅-C₆-Cycloalkyl, Phenyl, Naphthyl und einem 5- oder 6-gliedrigen heterocyclischen aromatischen Rest mit 1 bis 3 Heteroatomen, ausgewählt unter O, N und S,
A und B die in einem der Ansprüche 1 bis 3 angegebenen Bedeutungen besitzen,
wobei Ar nicht für Phenyl steht, wenn A eine Gruppe NR⁴ umfasst,
und die Salze davon mit physiologisch verträglichen Säuren,
ausgenommen die Verbindungen der Formel 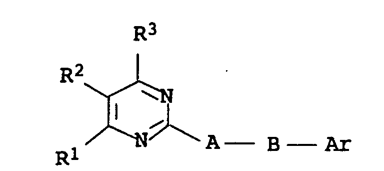 worin R¹ für OH oder SH steht, R² und R³ unabhängig voneinander für H, C₁-C₆-Alkyl, OC₁-C₆-Alkyl, SC₁-C₆-Alkyl, OH, SH oder NR⁴R⁵ stehen, wobei R⁴ und R⁵ für H oder C₁-C₆-Alkyl stehen, A für SC₁-C₆-Alkylen,
B für 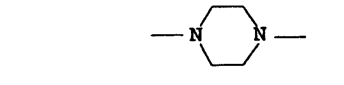 steht und Ar für Phenyl steht, das gegebenenfalls einen oder mehrere Substituenten aufweist, die ausgewählt sind unter C₁-C₄-Alkyl, OC₁-C₄-Alkyl, CF₃, F, Cl oder Br.

5. Verbindungen der Formel IB nach Anspruch 4, wobei B für 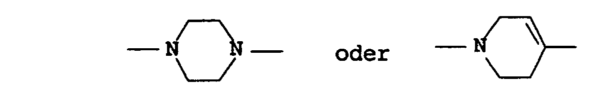 steht.

6. Verbindungen der Formel I nach Anspruch 4 oder 5, wobei R¹ für H, C₁-C₈-Alkyl, das gegebenenfalls durch OH, OC₁-C₈-Alkyl oder Halogen substituiert ist, OR⁴, SR⁴ oder NR⁴R⁵ steht, wobei R⁴ und R⁵ unabhängig voneinander für H oder C₁-C₈-Alkyl stehen; R² für H, OR⁴ oder C₁-C₈-Alkyl steht; und R³ für H steht.

7. Verbindungen der Formel IC: 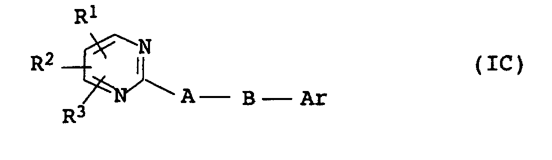 worin Ar für Phenyl steht, das gegebenenfalls ein bis vier Substituenten aufweist, die unabhängig voneinander ausgewählt sind unter C₁-C₈-Alkyl, das gegebenenfalls durch OH, OC₁-C₈-Alkyl oder Halogen substituiert ist, Phenyl, Naphthyl, Pyrrolyl, CN, NO₂, CF₃, CHF₂, Halogen, SO₂R⁴ oder SR⁴ steht, wobei R⁴ für H oder C₁-C₈-Alkyl steht und R¹, R², R³, A und B die in einem der Ansprüche 1 bis 6 angegebenen Bedeutungen besitzen, wobei A nicht die Gruppe NR⁴ umfasst, und die Salze davon mit physiologisch verträglichen Säuren, ausgenommen die Verbindungen der Formel 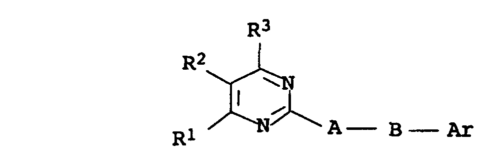 worin R¹ für OH oder SH steht, R² und R³ unabhängig voneinander für H, C₁-C₆-Alkyl, OC₁-C₆-Alkyl, SC₁-C₆-Alkyl, CO₂H, OH, SH, NR⁴R⁵ oder Halogen stehen, wobei R⁴ und R⁵ für H oder C₁-C₆-Alkyl stehen, A für SC₁-C₆-Alkylen,
B für 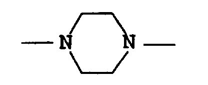 steht und Ar für Phenyl steht, das gegebenenfalls einen oder mehrere Substituenten aufweist, die ausgewählt sind unter C₁-C₄-Alkyl, SC₁-C₄-Alkyl, NO₂, CF₃, F, Cl oder Br.

8. Verbindungen nach Anspruch 7, wobei die Substituenten unabhängig voneinander ausgewählt sind unter C₁-C₈-Alkyl, Phenyl, CF₃, CHF₂, CN, NO₂, Halogen oder SR⁴ steht, wobei R⁴ für H oder C₁-C₈-Alkyl steht.

9. Verbindungen nach einem der vorhergehenden Ansprüche, wobei der Rest Ar einen oder zwei Substituenten aufweist, die sich jeweils in m-Stellung befinden.

10. Verbindungen nach einem der Ansprüche 1 bis 6, wobei
Ar für Pyridinyl steht, das gegebenenfalls ein bis vier Substituenten aufweist, die unabhängig voneinander ausgewählt sind unter C₁-C₈-Alkyl, Phenyl, Naphthyl, OH, OC₁-C₈-Alkyl, Halogen, CF₃, CN, C₂-C₆-Alkenyl, C₂-C₆-Alkinyl und einem 5- oder 6-gliedrigen, heterocyclischen, aromatischen Rest mit 1 bis 3 Heteroatomen, ausgewählt unter O, N und S.

11. Verbindungen nach einem der Ansprüche 1 bis 3, wobei
Ar für Pyrimidinyl steht, das gegebenenfalls ein bis drei Substituenten aufweist, die unabhängig voneinander ausgewählt sind unter C₁-C₈-Alkyl, Phenyl, C₅-C₆-Cycloalkyl, OH, OC₁-C₈-Alkyl, Halogen, CN, NO₂, CF₃, CHF₂ und einem 5-oder 6-gliedrigen, heterocyclischen, aromatischen oder nicht-aromatischen Rest mit 1 bis 3 Heteroatomen, ausgewählt unter O, N und S.

12. Verbindungen nach Anspruch 11, wobei
A für eine C₂-C₁₅-Alkylengruppe steht, die über S an den Pyrimidinring gebunden ist.

13. Verbindungen nach Anspruch 11 oder 12, wobei
Ar für 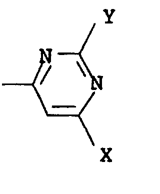 steht, worin X und Y unabhängig voneinander ausgewählt sind unter Halogen, CF₃, CHF₂, CN, NO₂, OH, C₁-C₈-Alkyl, OC₁-C₈-Alkyl und Phenyl.

14. Verbindungen nach einem der Ansprüche 11 bis 13, wobei R¹, R² und R³ unabhängig voneinander für H, C₁-C₈-Alkyl, NR⁴R⁵, SR⁴ oder OR⁴ stehen, wobei R⁴ und R⁵ unabhängig voneinander für H oder C₁-C₈-Alkyl stehen.

15. Verbindungen der Formel ID, 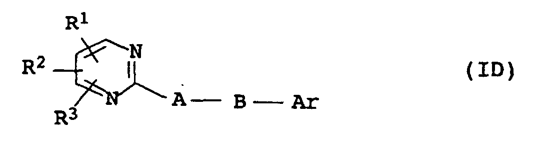 worin A für eine C₂-C₁₅-Alkylengruppe steht, die mindestens ein O-Atom umfasst, einer der Reste R¹, R² und R³ für OH steht und die beiden anderen der Reste R¹, R² und R³ sowie B und Ar die in einem der Ansprüche 1, 2, 4, 5, 7, 8, 9, 10, 11 oder 13 angegebenen Bedeutungen besitzen.

16. Verbindung nach Anspruch 1 der Formel 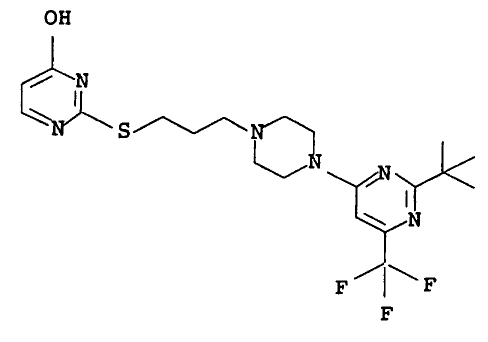 und die physiologisch verträglichen Salze davon.

17. Verbindungen der Formeln: 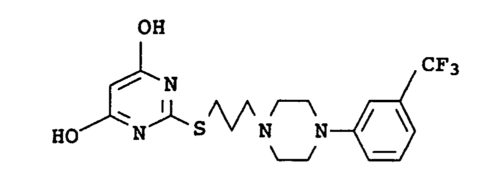 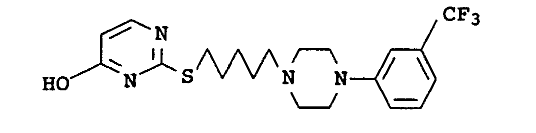 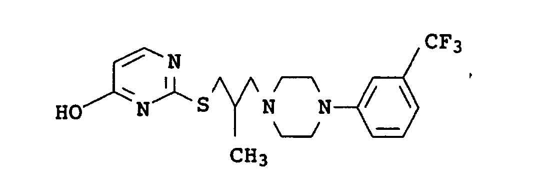 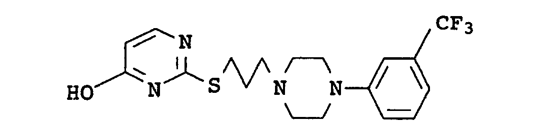 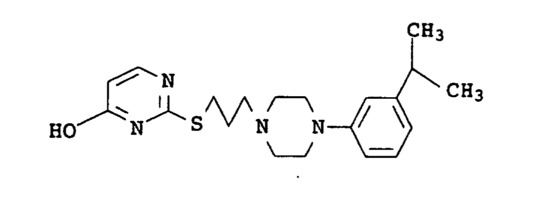 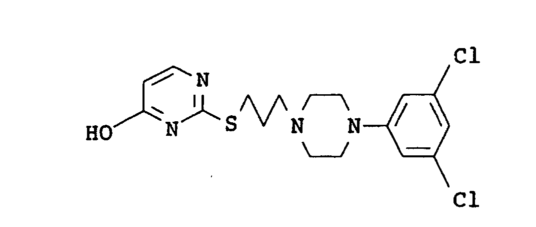 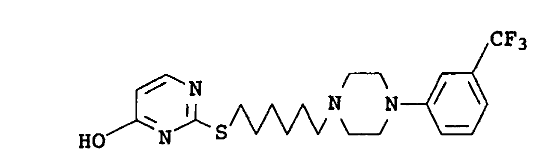 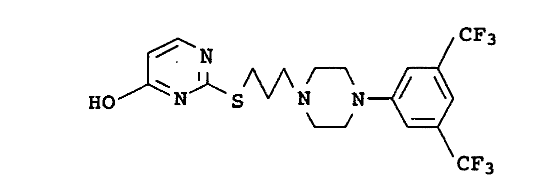 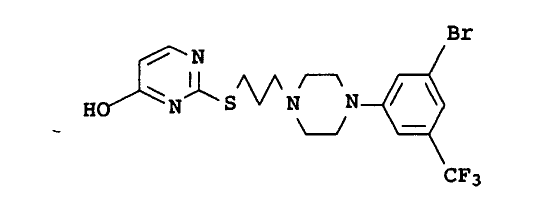 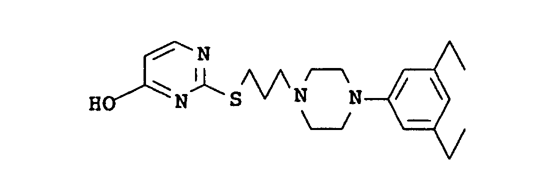 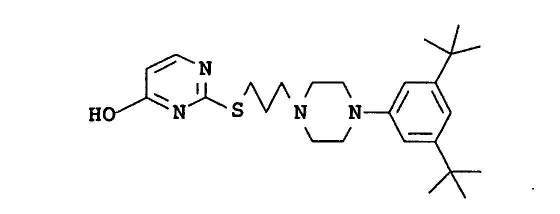 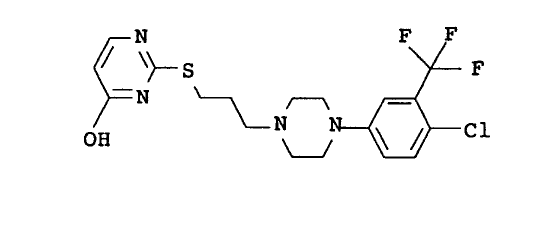 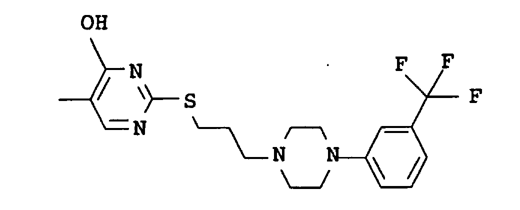 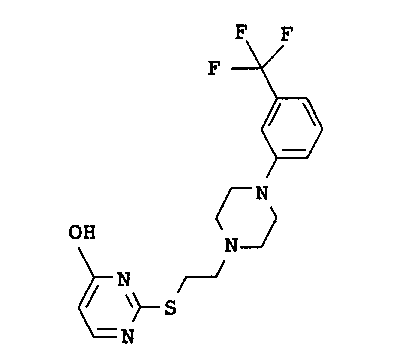 und die physiologisch verträglichen Salze davon.

18. Verfahren zur Herstellung der Verbindungen der Ansprüche 1 bis 17, **dadurch gekennzeichnet, daß** man
i) eine Verbindung der allgemeinen Formel II: 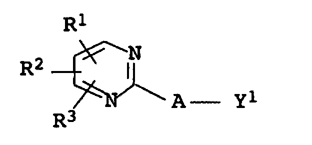 worin Y¹ für eine übliche Abgangsgruppe steht, mit einer Verbindung der allgemeinen Formel III
H - B - Ar
umsetzt;
ii) zur Herstellung einer Verbindung der Formel IA, IB oder IC, worin A ein Schwefelatom oder NR⁴ umfaßt, oder einer Verbindung der Formel ID:
eine Verbindung der allgemeinen Formel IV: 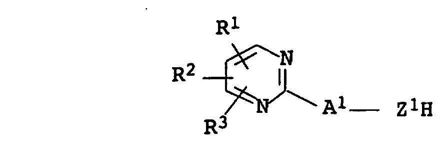
worin Z¹ für O oder S steht und A¹ für C₀-C₁₄-Alkylen steht, mit einer Verbindung der allgemeinen Formel VI
Y¹ - A² - B - Ar
worin Y¹ die oben angegebenen Bedeutungen besitzt, und A² für C₁-C₁₅-Alkylen steht, wobei A¹ und A² zusammen 2 bis 15 Kohlenstoffatome aufweisen, umsetzt,
iii) zur Herstellung einer Verbindung der Formel IA, IB oder IC, worin A die Gruppe COO oder CONR⁴ umfaßt:
eine Verbindung der allgemeinen Formel VII: 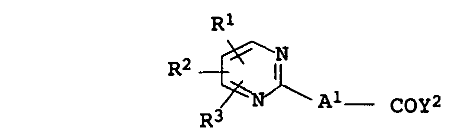
worin Y² für OH, OC₁-C₄-Alkyl, Cl oder zusammen mit CO für eine aktivierte Estergruppe steht, und A¹ die oben angegebenen Bedeutungen besitzt, mit einer Verbindung der Formel VIII:
Z¹ - A² - B - Ar
worin A² die oben angegebenen Bedeutungen besitzt, und Z¹ für OH oder NHR⁴ steht,
iv) zur Herstellung einer Verbindung der Formel IA, IB oder IC, worin A die Gruppe OCO oder NR⁴CO umfaßt:
eine Verbindung der Formel IV 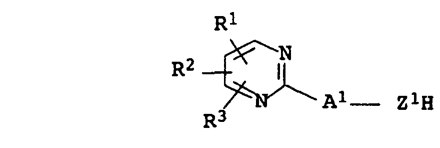
worin Z¹ für O oder NR⁴ steht, mit einer Verbindung der Formel X:
Y²CO - A² - B - Ar
worin A², B und Y² die oben angegebenen Bedeutungen besitzen, umsetzt, wobei in den obigen Formeln R¹, R², R³, A, B und Ar die in den Ansprüchen 1 bis 17 angegebenen Bedeutungen besitzen.

19. Pharmazeutisches Mittel, enthaltend mindestens eine Verbindung der Formeln IA, IB, IC oder ID nach einem der Ansprüche 1 bis 17, gegebenenfalls zusammen mit physioloisch akzeptablen Trägern und/oder Hilfsstoffen.

20. Verwendung einer Pyrimidinverbindung der Formel I: 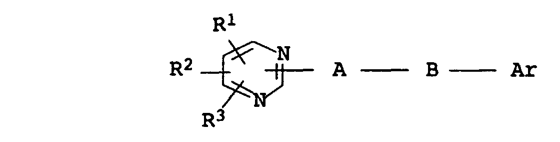 worin
A für eine C₁-C₁₈-Alkylengruppe steht, die gegebenenfalls mindestens eine Gruppe umfaßt, die ausgewählt ist unter O, S, NR⁴, CONR⁴, NR⁴CO, COO, OCO und einer Doppel- oder einer Dreifachbindung,
B für 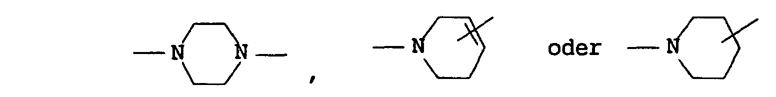 steht,
R¹, R², R³ unabhängig voneinander ausgewählt sind unter H, Halogen, OR⁴, NR⁴R⁵, SR⁴, CF₃, CN, CO₂R⁴ und C₁-C₈-Alkyl, das gegebenenfalls durch OH, OC₁-C₈-Alkyl oder Halogen substituiert ist,
R⁴ für H, C₁-C₈-Alkyl, das gegebenenfalls durch OH, OC₁-C₈-Alkyl oder Halogen substituiert ist, steht,
R⁵ die für R⁴ angegebenen Bedeutungen besitzt oder für COR⁴ oder CO₂R⁴ steht;
Ar für Phenyl, Pyridyl, Pyrimidyl oder Triazinyl steht,
wobei Ar gegebenenfalls ein bis vier Substituenten aufweisen kann, die unabhängig voneinander ausgewählt sind unter OR⁵, C₁-C₈-Alkyl, C₂-C₆-Alkenyl, C₂-C₆-Alkinyl, Halogen, CN, CO₂R⁴, NO₂, SO₂R⁴, SO₃R⁴, NR⁴R⁵, SO₂NR⁴R⁵, SR⁴, CF₃, CHF₂, einem 5- oder 6-gliedrigen carbocyclischen, aromatischen oder nicht-aromatischen Ring und einem 5-oder 6-gliedrigen heterocyclischen, aromatischen oder nicht-aromatischen Ring mit 1 bis 3 Heteroatomen, die ausgewählt sind unter 0, S und N,
wobei der Ring gegebenenfalls substituiert sein kann durch C₁-C₈-Alkyl, Hal, OC₁-C₈-Alkyl, OH, NO₂, CF₃ und
wobei
Ar gegebenenfalls auch mit einem carbocyclischen oder heterocyclischen Ring der oben definierten Art kondensiert sein kann,
und der Salze davon mit physiologisch verträglichen Säuren zur Herstellung eines pharmazeutischen Mittels zur Behandlung von Erkrankungen, die auf Dopamin-D₃-Rezeptorantagonisten bzw. -agonisten ansprechen.

## Claims

1. A pyrimidine compound of the formula IA: 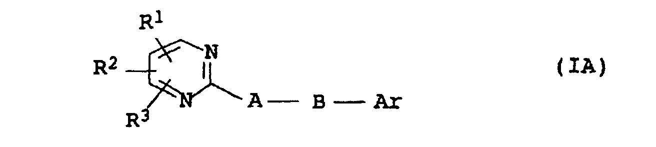 in which
A is a C₂-C₁₅-alkylene group which optionally comprises at least one group which is selected from S, NR⁴, CONR⁴, NR⁴CO, COO, OCO and a double or triple bond,
B is 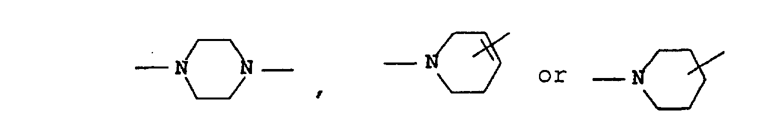
R¹, R², R³ are selected, independently of one another, from H, halogen, OR⁴, NR⁴R⁵, SR⁴, CF₃, CN, CO₂R⁴ and C₁-C₈-alkyl which is optionally substituted by OH, OC₁-C₈-alkyl or halogen,
R⁴ is H, C₁-C₈-alkyl which is optionally substituted by OH, OC₁-C₈-alkyl or halogen,
R⁵ has the meanings indicated for R⁴ or is COR⁴ or CO₂R⁴;
Ar is phenyl, pyridyl, pyrimidyl or triazinyl,
it being possible for Ar optionally to have one to four substituents which are selected, independently of one another, from OR⁵, C₁-C₈-alkyl, C₂-C₆-alkenyl, C₂-C₆-alkynyl, halogen, CN, CO₂R⁴, NO₂, SO₂R⁴, SO₃R⁴, NR⁴R⁵, SO₂NR⁴R⁵, SR⁴, CF₃, CHF₂, a 5- or 6-membered carbocyclic, aromatic or nonaromatic ring and a 5- or 6-membered heterocyclic aromatic or nonaromatic ring with 1 to 3 heteroatoms which are selected from O, S and N, it being possible for the ring optionally to be substituted by C₁-C₈-alkyl, Hal, OC₁-C₈-alkyl, OH, NO₂, CF₃ and
it being possible for Ar optionally also to be fused to a 5- or 6-membered carbocyclic, aromatic or nonaromatic ring or a 5- or 6-membered, aromatic or nonaromatic heterocyclic ring with 1 to 3 heteroatoms which are selected from O, S and N,
where Ar is not phenyl if A comprises an NR⁴ group,
and the salts thereof with physiologically tolerated acids,
except the compounds of the formula 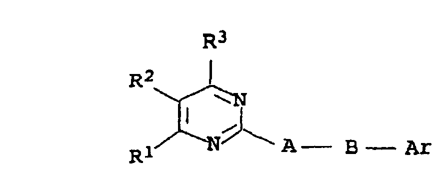 in which R¹ is OH or SH, R² and R³ are, independently of one another, H, C₁-C₆-alkyl, OC₁-C₆-alkyl, SC₁-C₆-alkyl, CO₂H, OH, SH, NR⁴R⁵ or halogen, where R⁴ and R⁵ are H or C₁-C₆-alkyl, A is SC₁-C₆-alkylene,
B is 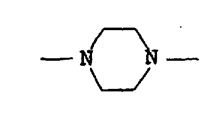 and Ar is phenyl which optionally has one or more substituents which are selected from C₁-C₄-alkyl, OC₁-C₄-alkyl, SC₁-C₄-alkyl, NO₂, CF₃, F, Cl or Br.

2. A pyrimidine compound of the formula IA as claimed in claim 1, where
Ar is phenyl, pyridyl, pyrimidyl or triazinyl, it being possible for Ar optionally to have one or two substituents X and Y which are selected, independently of one another, from OR⁵, C₁-C₈-alkyl, Hal, CN, CO₂R⁴, NO₂, SO₂R⁴, SO₃R⁴, NR⁴R⁵, SO₂NR⁴R⁵, SR⁴, CF₃, CHF₂, a 5- or 6-membered carbocyclic, aromatic or nonaromatic ring and a 5- or 6-membered heterocyclic aromatic or nonaromatic ring with 1 to 3 heteroatoms which are selected from O, S and N, it being possible for the ring optionally to be substituted by C₁-C₈-alkyl, Hal, OC₁-C₈-alkyl, OH, NO₂, CF₃ and
it being possible for Ar optionally also to be fused to a 5- or 6-membered carbocyclic, aromatic or nonaromatic ring or a 5- or 6-membered, aromatic or nonaromatic or heterocyclic ring with 1 to 3 heteroatoms which are selected from O, S and N.

3. A compound of the formula IA as claimed in claim 1 or 2, where A is C₃-C₁₀-alkylene which optionally comprises at least one group which is selected from S and a double or triple bond.

4. A compound of the formula 1B 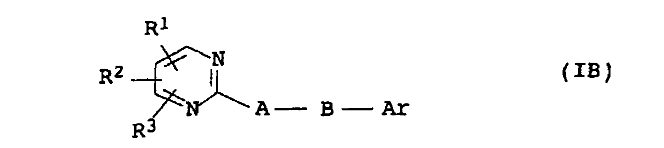 in which R¹, R² and R³ are, independently of one another, H, C₁-C₈-alkyl which may optionally be substituted by OH, OC₁-C₈-alkyl or halogen, or OH, OC₁-C₈-alkyl, SR⁴ or NR⁴R⁵, where R⁴ and R⁵ are, independently of one another, H or C₁-C₈-alkyl;
Ar is phenyl, pyridyl, or pyrimidyl, which optionally has one, two, three or four substituents which are selected from C₁-C₈-alkyl which is optionally substituted by OH, OC₁-C₈-alkyl or halogen, or OR^{4a} where R^{4a} is H, C₁-C₈-alkyl which is optionally substituted by OH, OC₁-C₈-alkyl or halogen, or CHF₂, CF₃, CN, halogen, C₂-C₆-alkenyl, C₂-C₆-alkynyl, C₅-C₆-cycloalkyl, phenyl, naphthyl and a 5- or 6-membered heterocyclic aromatic radical with 1 to 3 heteroatoms selected from O, N and S,
A and B have the meanings indicated in any of claims 1 to 3,
where Ar is not phenyl, if A comprises an NR⁴ group,
and the salts thereof with physiologically tolerated acids,
except the compounds of the formula 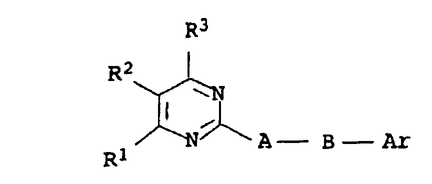 in which R¹ is OH or SH, R² and R³ are, independently of one another, H, C₁-C₆-alkyl, OC₁-C₆-alkyl, SC₁-C₆-alkyl, OH, SH or NR⁴R⁵, where R⁴ and R⁵ are H or C₁-C₆-alkyl, A is SC₁-C₆-alkylene,
B is 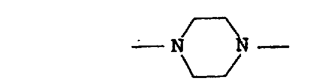 and Ar is phenyl which optionally has one or more substituents which are selected from C₁-C₄-alkyl, OC₁-C₄-alkyl, CF₃, F, Cl or Br.

5. A compound of the formula IB as claimed in claim 4, where B is

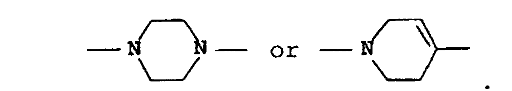

6. A compound of the formula I as claimed in claim 4 or 5, where R¹ is H, C₁-C₈-alkyl which is optionally substituted by OH, OC₁-C₈-alkyl or halogen, or OR⁴, SR⁴ or NR⁴R⁵, where R⁴ and R⁵ are, independently of one another, H or C₁-C₈-alkyl; R² is H, OR⁴ or C₁-C₈-alkyl; and R³ is H.

7. A compound of the formula IC: 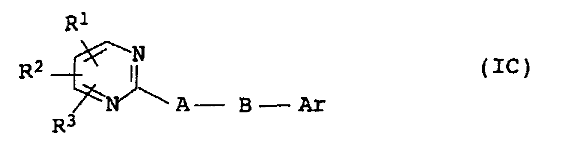 in which Ar is phenyl which optionally has one to four substituents which are selected, independently of one another, from C₁-C₈-alkyl which is optionally substituted by OH, OC₁-C₈-alkyl or halogen, or phenyl, naphthyl, pyrrolyl, CN, NO₂, CF₃, CHF₂, halogen, SO₂R⁴ or SR⁴, where R⁴ is H or C₁-C₈-alkyl, and R¹, R², R³, A and B have the meanings indicated in any of claims 1 to 6, where A does not comprise the NR⁴ group, and the salts thereof with physiologically tolerated acids, except the compounds of the formula 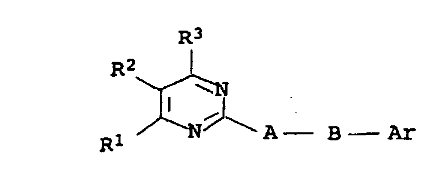 in which R¹ is OH or SH, R² and R³ are, independently of one another, H, C₁-C₆-alkyl, OC₁-C₆-alkyl, SC₁-C₆-alkyl, CO₂H, OH, SH, NR⁴R⁵ or halogen, where R⁴ and R⁵ are H or C₁-c₆-alkyl, A is SC₁-C₆-alkylene,
B is 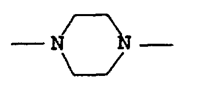 and Ar is phenyl
which optionally has one or more substituents which are selected from C₁-C₄-alkyl, SC₁-C₄-alkyl, NO₂, CF₃, F, Cl or Br.

8. A compound as claimed in claim 7, where the substituents are selected, independently of one another, from C₁-C₈-alkyl, phenyl, CF₃, CHF₂, CN, NO₂, halogen or SR⁴, where R⁴ is H or C₁-C₈-alkyl.

9. A compound as claimed in any of the preceding claims, where the Ar radical has one or two substituents which are each in an m position.

10. A compound as claimed in any of claims 1 to 6, wherein
Ar is pyridinyl which optionally has one to four substituents which are selected, independently of one another, from C₁-C₈-alkyl, phenyl, naphthyl, OH, OC₁-C₈-alkyl, halogen CF₃, CN, C₂-C₆-alkenyl, C₂-C₆-alkynyl and a 5- or 6-membered heterocyclic aromatic radical with 1 to 3 heteroatoms selected from O, N and S.

11. A compound as claimed in any of claims 1 to 3, where
Ar is pyrimidinyl which optionally has one to three substituents which are selected, independently of one another, from C₁-C₈-alkyl, phenyl, C₅-C₆-cycloalkyl, OH, OC₁-C₈-alkyl, halogen, CN, NO₂, CF₃, CHF₂ and a 5- or 6-membered heterocyclic aromatic or nonaromatic radical with 1 to 3 heteroatoms selected from O, N and S.

12. A compound as claimed in claim 11, where
A is a C₂-C₁₅-alkylene group which is linked via S to the pyrimidine ring.

13. A compound as claimed in claim 11 or 12, where
Ar is 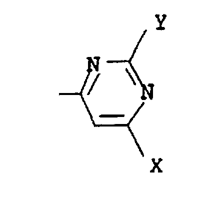 in which X and Y are selected, independently of one another, from halogen, CF₃, CHF₂, CN, NO₂, OH, C₁-C₈-alkyl, OC₁-C₆-alkyl and phenyl.

14. A compound as claimed in any of claims 11 to 13, where R¹, R² and R³ are, independently of one another, H, C₁-C₈-alkyl, NR⁴R⁵, SR⁴ or OR⁴, where R⁴ and R⁵ are, independently of one another, H or C₁-C₈-alkyl.

15. A compound of the formula ID, 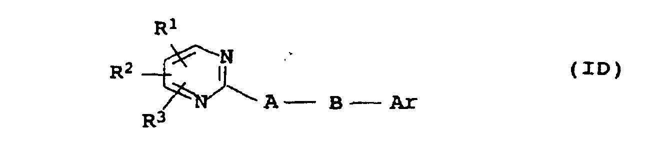 in which A is a C₂-C₁₅-alkylene group which comprises at least one O atom, one of the radicals R¹, R² and R³ is OH and the other two of the radicals R¹, R² and R³, B and Ar have the meanings indicated in any of claims 1, 2, 4, 5, 7, 8, 9, 10, 11 or 13.

16. A compound as claimed in claim 1 of the formula 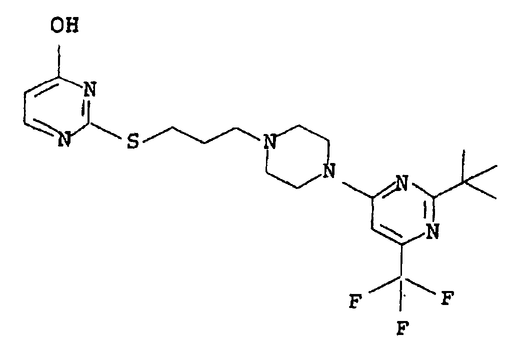 and the physiologically tolerated salts thereof.

17. A compound of the formula 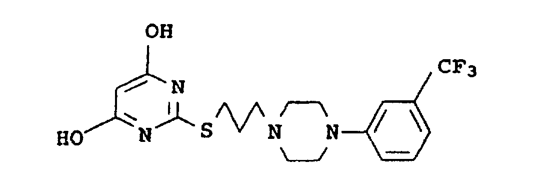 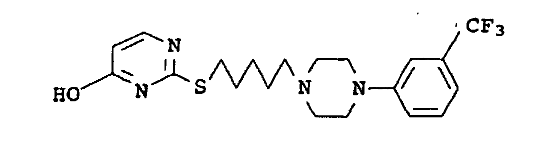 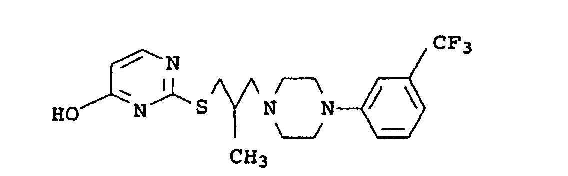 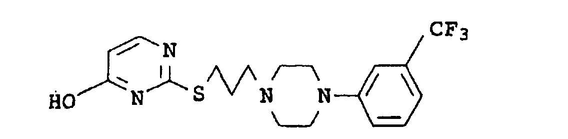 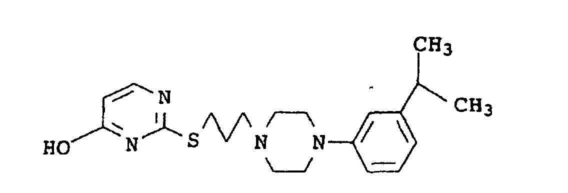 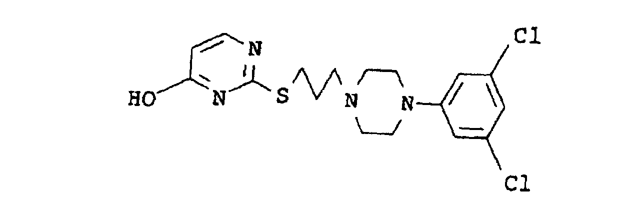 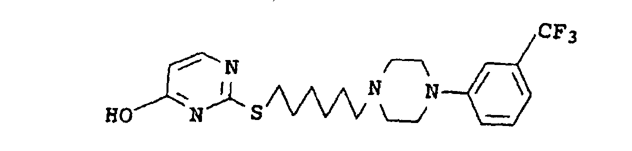 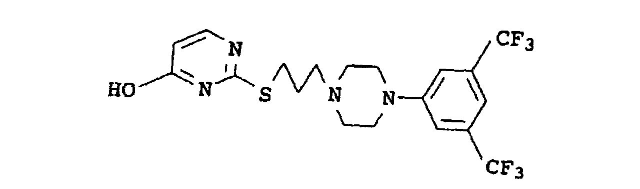 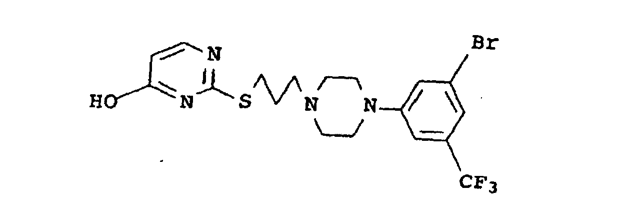 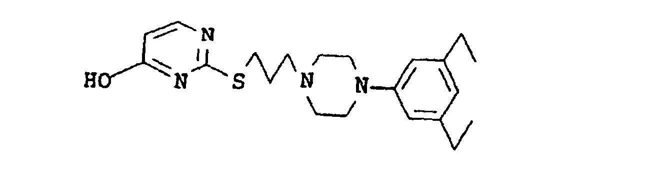 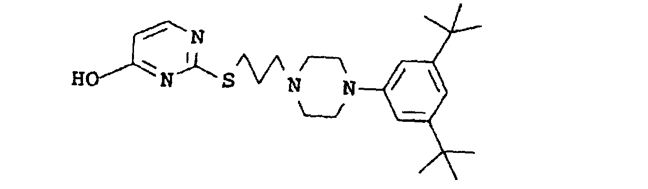 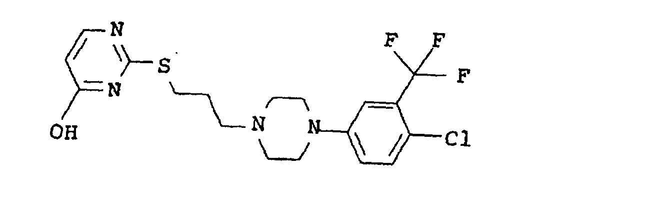 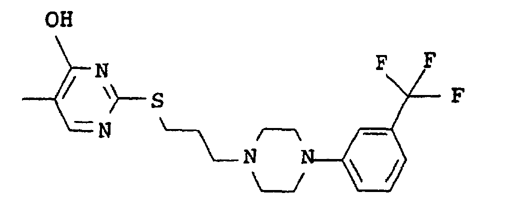 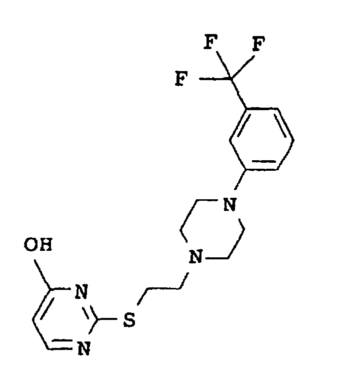 and the physiologically tolerated salts thereof.

18. A process for preparing the compounds of claims 1 to 17, which comprises
i) reacting a compound of the general formula II: 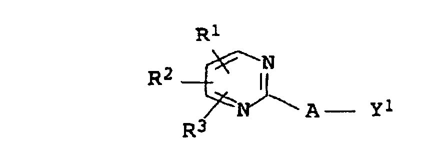 in which Y¹ is a conventional leaving group, with a compound of the general formula III
H - B - Ar;
ii) to prepare a compound of the formula IA, IB or IC in which A comprises a sulfur atom or NR⁴, or a compound of the formula ID:
reacting a compound of the general formula IV: 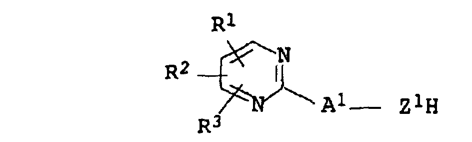 in which Z¹ is O or S, and A¹ is C₀-C₁₄-alkylene, with a compound of the general formula VI
Y¹ - A² - B - Ar
in which Y¹ has the meanings indicated above, and A² is C₁-C₁₅-alkylene, where A¹ and A² together have 2 to 15 carbon atoms,
iii) to prepare a compound of the formula IA, IB or IC in which A comprises the group COO or CONR⁴:
reacting a compound of the general formula VII: 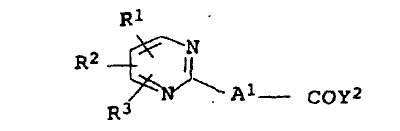 in which Y² is OH, OC₁-C₄-alkyl, Cl or together with CO is an activated ester group, and A¹ has the meanings indicated above, with a compound of the formula VIII:
Z¹ - A² - B - Ar
in which A² has the meanings indicated above, and Z¹ is OH or NHR⁴,
iv) to prepare a compound of the formula IA, IB or IC in which A comprises the group OCO or NR⁴CO:
reacting a compound of the formula IV 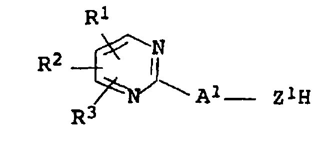 in which Z¹ is O or NR⁴, with a compound of the formula X:
Y²CO - A² - B - Ar
in which A², B and Y² have the meanings indicated above, where R¹, R², R³, A, B and Ar in the above formulae have the meanings indicated in claims 1 to 17.

19. A pharmaceutical composition comprising at least one compound of the formulae IA, IB, IC or ID as claimed in any of claims 1 to 17, where appropriate together with physiologically acceptable carriers and/or excipients.

20. The use of a pyrimidine compound of the formula I: 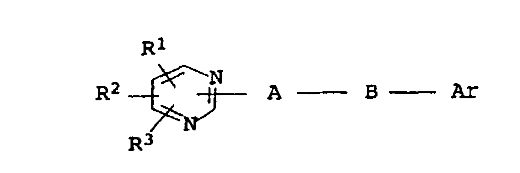 in which
A is a C₁-C₁₈-alkylene group which optionally comprises at least one group which is selected from O, S, NR⁴, CONR⁴, NR⁴CO, COO, OCO and a double or triple bond,
B is 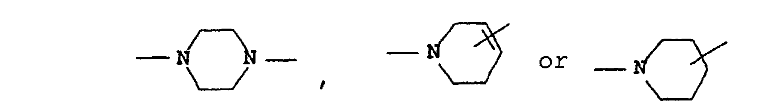
R¹ R², R³ are selected, independently of one another, from H, halogen, OR⁴, NR⁴R⁵, SR⁴, CF₃, CN, CO₂R⁴ and C₁-C₈-alkyl which is optionally substituted by OH, OC₁-C₈-alkyl or halogen,
R⁴ is H, C₁-C₈-alkyl which is optionally substituted by OH, OC₁-C₈-alkyl or halogen,
R⁵ has the meanings indicated for R⁴ or is COR⁴ or CO₂R⁴;
Ar is phenyl, pyridyl, pyrimidyl or triazinyl,
it being possible for Ar optionally to have one to four substituents which are selected, independently of one another, from OR⁵, C₁-C₈-alkyl, C₂-C₆-alkenyl, C₂-C₆-alkynyl, halogen, CN, CO₂R⁴, NO₂, SO₂R⁴, SO₃R⁴, NR⁴R⁵, SO₂NR⁴R⁵, SR⁴, CF₃, CHF₂, a 5- or 6-membered carbocyclic, aromatic or nonaromatic ring and a 5- or 6-membered heterocyclic aromatic or nonaromatic ring with 1 to 3 heteroatoms which are selected from O, S and N,
it being possible for the ring optionally to be substituted by C₁-C₈-alkyl, Hal, OC₁-C₈-alkyl, OH, NO₂, CF₃ and
it being possible for Ar optionally also to be fused to a carbocyclic or heterocyclic ring of the type defined above,
and the salts thereof with physiologically tolerated acids for preparing a pharmaceutical composition for the treatment of disorders which respond to dopamine D₃ receptor antagonists or agonists.

## Revendications

1. Composés de pyrimidine de formule IA: 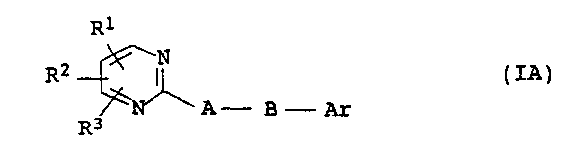 où
A désigne un groupe alkylène en C₂-C₁₅, qui inclut éventuellement au moins un groupe qui est choisi parmi S, NR⁴, CONR⁴, NR⁴CO, COO, OCO et une double ou triple liaison,
B représente 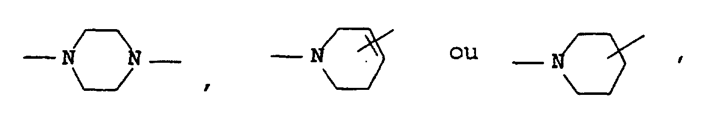
R¹, R², R³ sont choisis indépendamment l'un de l'autre parmi H, halogéno, OR⁴, NR⁴R⁵, SR⁴, CF₃, CN, CO₂R⁴ et alkyle en C₁-C₈, qui est éventuellement substitué par OH, O-alkyle en C₁-C₈ ou halogéno,
R⁴ désigne H ou un groupe alkyle en C₁-C₈, qui est éventuellement substitué par un groupe OH, O-alkyle en C₁-C₈ ou halogéno,
R⁵ possède les significations indiquées pour R⁴ ou représente un groupe COR⁴ ou CO₂R⁴,
Ar désigne un groupe phényle, pyridyle, pyrimidinyle ou triazinyle,
tandis que Ar peut présenter éventuellement un à quatre substituants, qui sont choisis indépendamment l'un de l'autre parmi les groupes OR⁵, alkyle en C₁-C₈, alcényle en C₂-C₆, alcynyle en C₂-C₆, halogéno, CN, CO₂R⁴, NO₂, SO₂R⁴, SO₃R⁴, NR⁴R⁵, SO₂NR⁴R⁵, SR⁴, CF₃, CHF₂, un cycle carbocyclique, aromatique ou non-aromatique à 5 ou 6 chaînons, et un cycle hétérocyclique, aromatique ou non-aromatique, à 5 ou 6 chaînons, ayant 1 à 3 hétéroatomes, qui sont choisis parmi O, S et N, tandis que le cycle peut être éventuellement substitué par un groupe alkyle en C₁-C₈, halogéno, O-alkyle en C₁-C₈, OH, NO₂, CF₃, et
tandis que Ar peut également être éventuellement condensé avec un cycle carbocyclique, aromatique ou non-aromatique, ayant 5 ou 6 chaînons, ou un cycle hétérocyclique, aromatique ou non-aromatique, à 5 ou 6 chaînons ayant 1 à 3 hétéroatomes, qui sont choisis parmi O, S et N,
tandis que Ar ne désigne pas le groupe phényle lorsque A englobe un groupe NR⁴,
et leurs sels avec des acides physiologiquement acceptables,
à l'exception des composés de formule 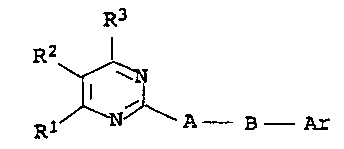 où R¹ désigne OH ou SH, R² et R³ représentent indépendamment l'un de l'autre un groupe H, alkyle en C₁-C₆, O-alkyle en C₁-C₆, S-alkyle en C₁-C₆, CO₂H, OH, SH, NR⁴R⁵ ou halogéno, tandis que R⁴ et R⁵ représentent H ou un alkyle en C₁-C₆, A désigne un groupe S-alkylène en C₁-C₆,
B désigne 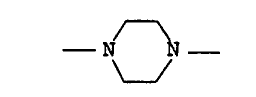 et Ar représente un groupe phényle, qui présente éventuellement un ou plusieurs substituants qui sont choisis parmi les groupes alkyle en C₁-C₄, O-alkyle en C₁-C₄, S-alkyle en C₁-C₄, NO₂, CF₃, F, Cl ou Br.

2. Composés de pyrimidine de formule IA selon la revendication 1, dans lesquels
Ar désigne un groupe phényle, pyridyle, pyrimidyle
ou triazinyle, tandis que Ar peut éventuellement présenter un ou deux substituants X et Y, qui sont choisis indépendamment l'un de l'autre parmi OR⁵, alkyle en C₁-C₈, halogéno, CN, CO₂R⁴, NO₂, SO₂R⁴, SO₃R⁴, NR⁴R⁵, SO₂NR⁴R⁵, SR⁴, CF₃, CHF₂, un cycle carbocyclique, aromatique ou non-aromatique à 5 ou 6 chaînons, et un cycle hétérocyclique à 5
ou 6 chaînons, aromatique ou non-aromatique, ayant 1 à 3 hétéroatomes qui sont choisis parmi O, S et N, tandis que le cycle peut être éventuellement substitué par un groupe alkyle en C₁-C₈, halogéno, O-alkyle en C₁-C₈, OH, NO₂, CF₃, et tandis que
Ar peut également être éventuellement condensé avec un cycle carbocyclique, aromatique ou non-aromatique, ayant 5 ou 6 chaînons, ou un cycle hétérocyclique, aromatique ou non-aromatique, à 5
ou 6 chaînons ayant 1 à 3 hétéroatomes, qui sont choisis parmi O, S et N.

3. Composés de formule IA selon la revendication 1 ou 2, dans lesquels A désigne un groupe alkylène en C₃-C₁₀, qui englobe éventuellement au moins un groupe qui est choisi parmi S et une double ou triple liaison.

4. Composés de formule IB 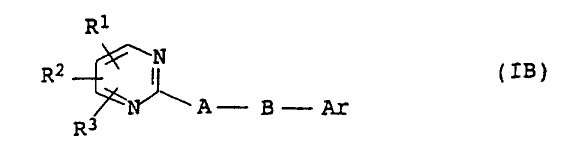 où R¹, R² et R³ représentent, indépendamment l'un de l'autre, H, alkyle en C₁-C₈ - qui peut être éventuellement substitué par OH, O-alkyle en C₁-C₈ ou halogéno -, OH, O-alkyle en C₁-C₈, SR⁴ ou NR⁴R⁵, tandis que R⁴et R⁵ désignent, indépendamment l'un de l'autre, H ou alkyle en C₁-C₈;
Ar désigne un phényle, pyridyle ou pyrimidyle, qui présente éventuellement un, deux, trois ou quatre substituants, qui sont choisis parmi les groupes alkyle en C₁-C₈ - qui est éventuellement substitué par un groupe OH, O-alkyle en C₁-C₈ ou halogéno -, OR^{4a}, tandis que R^{4a} désigne un groupe H, alkyle en C₁-C₈ - qui est éventuellement substitué par OH, O-alkyle en C₁-C₈ ou halogéno-, CHF₂, CF₃, CN, halogéno, alcényle en C₂-C₆, alcynyle en C₂-C₆, cycloalkyle en C₅-C₆, phényle, naphtyle et un reste aromatique hétérocyclique à 5 ou 6 chaînons ayant 1 à 3 hétéroatomes, choisis parmi O, N et S,
A et B ont les significations indiquées dans l'une des revendications 1 à 3,
tandis que Ar ne désigne pas un phényle, lorsque A englobe un groupe NR⁴,
et leurs sels avec des acides physiologiquement acceptables, à l'exception des composés de formule 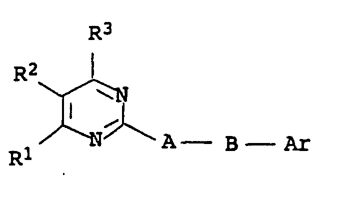 où R¹ représente OH ou SH, R² et R³ désignent indépendamment l'un de l'autre H, alkyle en C₁-C₆, O-alkyle en C₁-C₆, S-alkyle en C₁-C₆, OH, SH ou NR⁴R⁵, tandis que R⁴ et R⁵ désignent H ou alkyle en C₁-C₆, A représente un S-alkylène en C₁-C₆,
B désigne 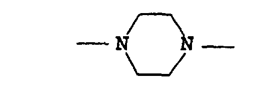 et
Ar représente un phényle, qui présente éventuellement un ou plusieurs substituants, qui sont choisis parmi les groupes alkyle en C₁-C₄, O-alkyle en C₁-C₄, CF₃, F, Cl ou Br.

5. Composés de formule IB selon la revendication 4, dans lesquels B représente

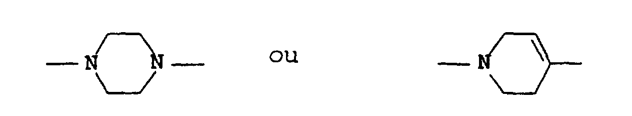

6. Composés de formule I selon la revendication 4 ou 5, dans lesquels R¹ désigne H, alkyle en C₁-C₈ - qui est éventuellement substitué par OH, O-alkyle en C₁-C₈ ou halogéno -, OR⁴, SR⁴ ou NR⁴R⁵, tandis que R⁴ et R⁵ désignent, indépendamment l'un de l'autre, H ou alkyle en C₁-C₈; R² désigne H, OR⁴ ou alkyle en C₁-C₈; et R³ désigne H.

7. Composés de formule IC: 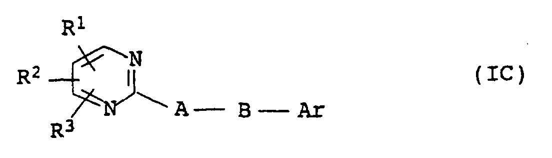 où Ar représente un phényle, qui présente éventuellement un à quatre substituants, qui sont choisis indépendamment l'un de l'autre parmi les groupes alkyle en C₁-C₈ - qui est éventuellement substitué par OH, O-alkyle en C₁-C₈ ou halogéno -, phényle, naphtyle, pyrrolyle, CN, NO₂, CF₃, CHF₂, halogéno, SO₂R⁴ ou SR⁴, tandis que R⁴ désigne H ou alkyle en C₁-C₈, et R¹, R², R³, A et B possèdent les significations indiquées dans l'une des revendications 1 à 6, tandis que A n'englobe pas le groupe NR⁴, et leurs sels avec des acides physiologiquement acceptables, à l'exception des composés de formule 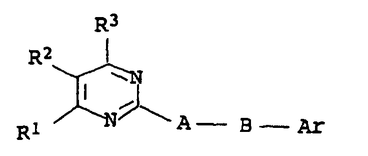 où R¹ désigne OH ou SH, R² et R³ représentent, indépendamment l'un de l'autre, H, alkyle en C₁-C₆, O-alkyle en C₁-C₆, S-alkyle en C₁-C₆, CO₂H, OH, SH, NR⁴R⁵ ou halogéno, tandis que R⁴ et R⁵ désignent H ou alkyle en C₁-C₆, A représente S-alkylène en C₁-C₆,
B désigne 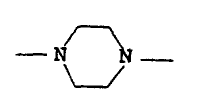 et
Ar représente un phényle, qui présente éventuellement un ou plusieurs substituants, qui sont choisis parmi alkyle en C₁-C₄, S-alkyle en C₁-C₄, NO₂, CF3, F, Cl ou Br.

8. Composés selon la revendication 7, dans lesquels les substituants sont choisis indépendamment l'un de l'autre parmi les groupes alkyle en C₁-C₈, phényle, CF₃, CHF₂, CN, NO₂, halogéno ou SR⁴, tandis que R⁴ désigne H ou alkyle en C₁-C₈.

9. Composés selon l'une des revendications précédentes, dans lesquels le reste Ar présente un ou deux substituants, qui se trouvent à chaque fois en position m.

10. Composés selon l'une des revendications 1 à 6, dans lesquels
Ar désigne un pyridinyle, qui présente éventuellement un à quatre substituants qui sont choisis indépendamment l'un de l'autre parmi alkyle en C₁-C₈, phényle, naphtyle, OH, O-alkyle en C₁-C₈, halogéno, CF₃, CN, alcényle en C₂-C₆, alcynyle en C₂-C₆ et un reste aromatique, hétérocyclique à 5
ou 6 chaînons, ayant 1 à 3 hétéroatomes, choisis parmi O, N et S.

11. Composés selon l'une des revendications 1 à 3, dans lesquels
Ar désigne un pyrimidinyle, qui présente éventuellement un à trois substituants qui sont choisis indépendamment l'un de l'autre parmi alkyle en C₁-C₈, phényle, cycloalkyle en C₅-C₆, OH, O-alkyle en C₁-C₈, halogéno, CN, NO₂, CF₃, CHF₂ et un reste aromatique ou non-aromatique, hétérocyclique à 5 ou 6 chaînons, ayant 1 à 3 hétéroatomes, choisis parmi O, N et S.

12. Composés selon la revendication 11, dans lesquels
A représente un groupe alkylène en C₂-C₁₅, qui est lié au cycle pyrimidinyle par l'intermédiaire de S.

13. Composés selon la revendication 11 ou 12, dans lesquels
Ar désigne 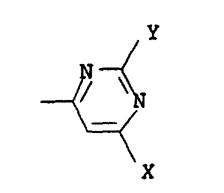 où X et Y sont choisis indépendamment l'un de l'autre parmi halogéno, CF₃, CHF₂, CN, NO₂, OH, alkyle en C₁-C₈, O-alkyle en C₁-C₈ et phényle.

14. Composés selon l'une des revendications 11 à 13, dans lesquels R¹, R² et R³ désignent indépendamment l'un de l'autre H, alkyle en C₁-C₈, NR⁴R⁵, SR⁴ ou OR⁴, tandis que R⁴ et R⁵ représentent indépendamment l'un de l'autre H ou alkyle en C₁-C₈.

15. Composés de formule ID, 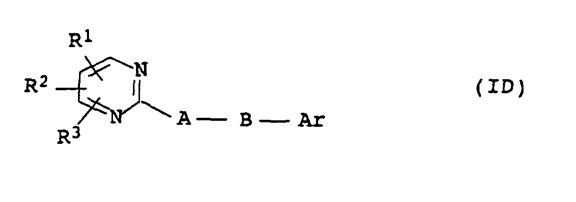 où A désigne un groupe alkylène en C₂-C₁₅, qui possède au moins un atome O, l'un des restes R¹, R² et R³ désigne OH et les deux autres des restes R¹, R² et R³, ainsi que B et Ar ont les significations indiquées dans les revendications 1, 2, 4, 5, 7, 8, 9, 10, 11 ou 13.

16. Composé selon la revendication 1 de formule 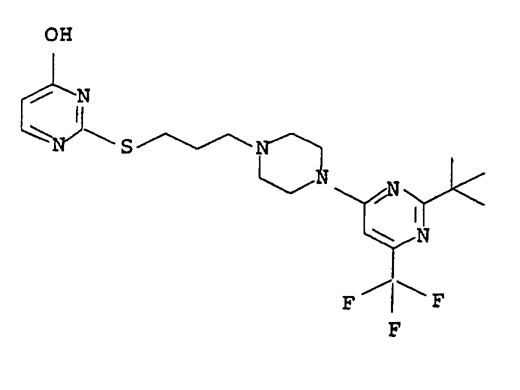 et ses sels physiologiquement acceptables.

17. Composés de formules: 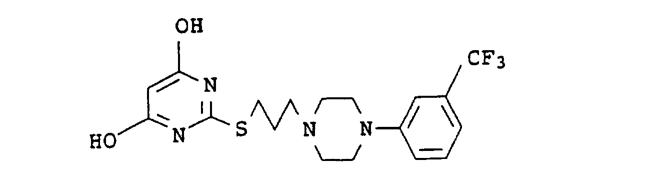 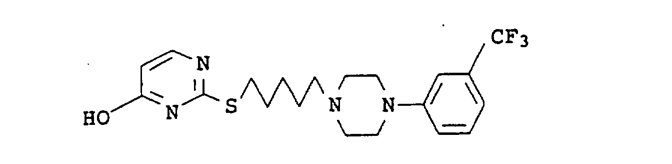 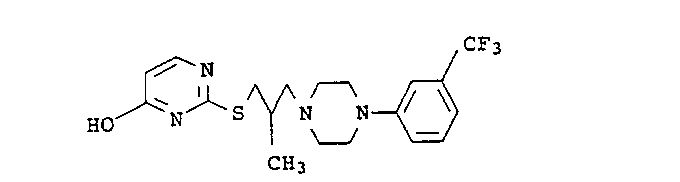 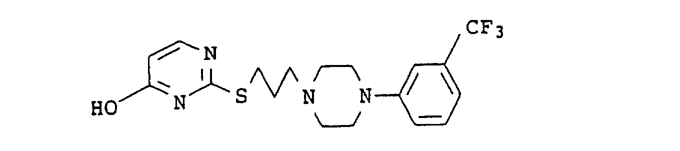 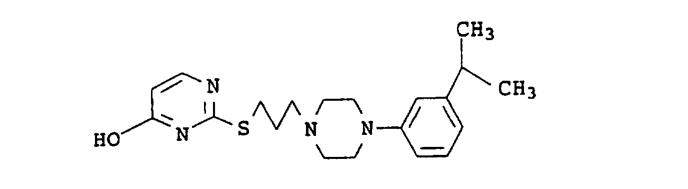 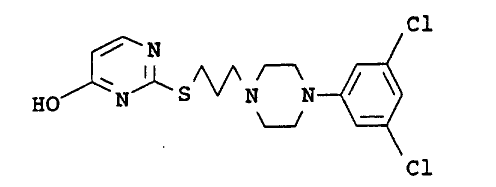 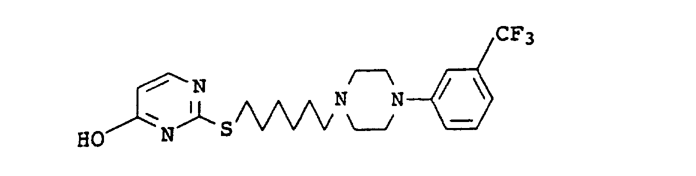 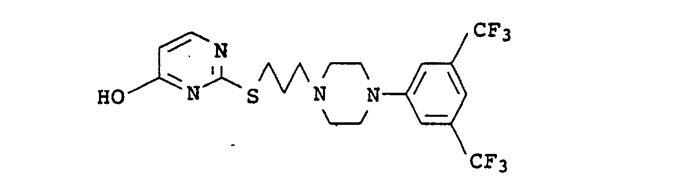 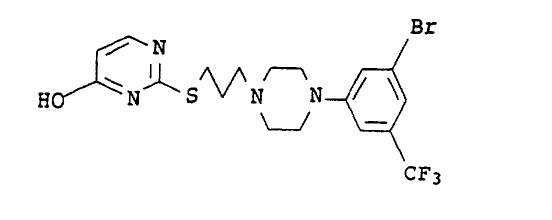 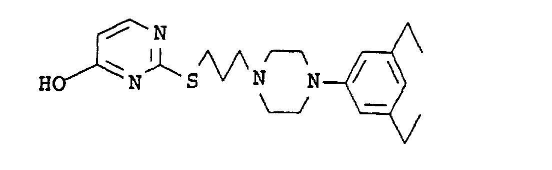 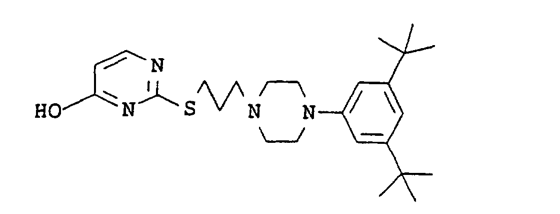 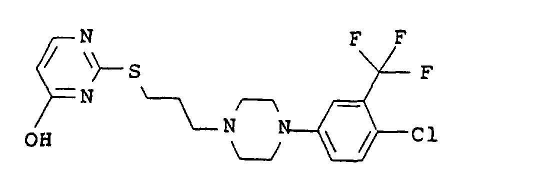 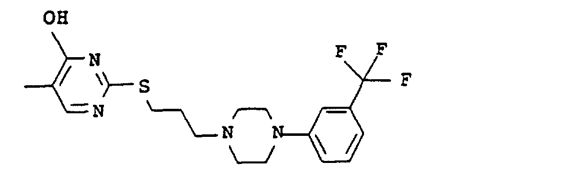 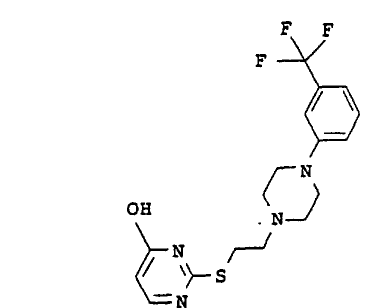 et ses sels physiologiquement acceptables.

18. Procédé pour la préparation des composés selon les revendications 1 à 17, **caractérisé par le fait qu'**on
i) fait réagir un composé de formule générale II: 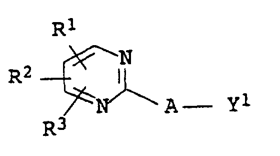 où Y¹ désigne un groupe séparable classique, avec un composé de formule générale III
H - B - Ar;
ii) pour la préparation d'un composé de formule IA, IB ou IC, où A englobe un atome de soufre ou NR⁴, ou d'un composé de formule ID:
fait réagir un composé de formule générale IV: 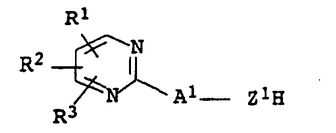
où Z¹ désigne O ou S, et A¹ représente un alkylène en C₀-C₁₄, avec un composé de formule générale VI
Y¹ - A² - B - Ar
où Y¹ possède les significations indiquées plus haut, et A² représente un alkylène en C₁-C₁₅, tandis que A¹ et A² présentent ensemble 2 à 15 atomes de carbone,
iii) pour la préparation d'un composé de formule IA, IB ou IC, où A englobe le groupe COO ou CONR⁴:
fait réagir un composé de formule générale VII: 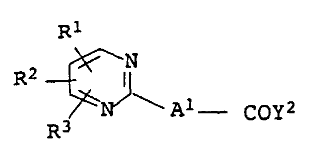
où Y² désigne OH, O-alkyle en C₁-C₄, C1 ou conjointement avec CO un groupe ester activé, et A¹ possède les significations indiquées plus haut, avec un composé de formule VIII:
Z¹ - A² - B - Ar
où A² possède les significations indiquées plus haut, et Z¹ représente OH ou NHR⁴,
iv) pour la préparation d'un composé de formule IA, IB ou IC, où A englobe le groupe OCO ou NR⁴CO:
fait réagir un composé de formule IV 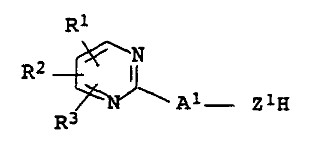
où Z¹ désigne O ou NR⁴, avec un composé de formule X:
Y²CO - A² - B - Ar
où A², B et Y² possèdent les significations indiquées plus haut, tandis que dans les formules susdites R¹, R², R³, A, B et Ar possèdent les significations indiquées dans les revendications 1 à 17.

19. Produit pharmaceutique, contenant au moins un composé de formules IA, IB, IC ou ID selon l'une des revendications 1 à 17, éventuellement conjointement avec des supports et/ou adjuvants physiologiquement acceptables.

20. Utilisation d'un composé de pyrimidine de formule I:
où 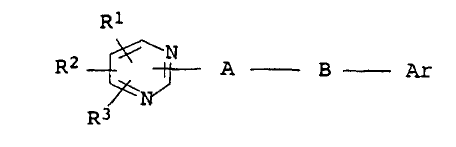
A représente un groupe alkylène en C₁-C₁₈, qui englobe éventuellement un groupe qui est choisi parmi O, S, NR⁴, CONR⁴, NR⁴CO, COO, OCO et une double ou une triple liaison,
B désigne 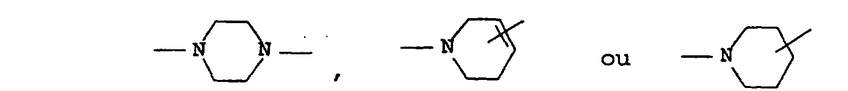
R¹, R², R³ désignent indépendamment l'un de l'autre H, halogéno, OR⁴, NR⁴R⁵, SR⁴, CF₃, CN, CO₂R⁴ et alkyle en C₁-C₈, qui est éventuellement substitué par un groupe OH, O-alkyle en C₁-C₈ ou halogéno,
R⁴ représente H, alkyle en C₁-C₈, qui est éventuellement substitué par OH, O-alkyle en C₁-C₈ ou halogéno,
R⁵ possède les significations indiquées pour R⁴ ou pour COR⁴ ou CO₂R⁴,
Ar représente un phényle, pyridyle, pyrimidyle ou triazinyle,
tandis que Ar peut éventuellement présenter un à quatre substituants, qui sont choisis indépendamment l'un de l'autre parmi OR⁵, alkyle en C₁-C₈, alcényle en C₂-C₆, alcynyle en C₂-C₆, halogéno, CN, CO₂R⁴, NO₂, SO₂R⁴, SO₃R⁴, NR⁴R⁵, SO₂NR⁴R⁵, SR⁴, CF₃, CHF₂, un cycle carbocyclique à 5 ou 6 chaînons, aromatique ou non-aromatique, et un cycle hétérocyclique à 5 ou 6 chaînons, aromatique ou non-aromatique, ayant 1 à 3 hétéroatomes, qui sont choisis parmi O, S et N,
tandis que le cycle peut éventuellement être substitué par alkyle en C₁-C₈, halogéno, O-alkyle en C₁-C₈, OH, NO₂, CF₃ et
tandis que
Ar peut éventuellement être également condensé avec un cycle carbocyclique ou hétérocyclique du type défini plus haut,
et leurs sels avec des acides physiologiquement acceptables pour la préparation d'un produit pharmaceutique pour le traitement d'affections qui répondent respectivement à des antagonistes ou des agonistes de récepteur D3 de la dopamine.
